(19) Europäisches Patentamt
European Patent Office
Office européen des brevets

(11) EP 1 531 161 A2

(12) EUROPEAN PATENT APPLICATION

(43) Date of publication:
18.05.2005 Bulletin 2005/20

(21) Application number: 04016076.4

(22) Date of filing: 27.07.1999

(51) Int Cl.⁷: **C07K 14/47**, C12N 15/12, C12N 5/10, G01N 33/50, C12Q 1/68, C07K 16/18, A61K 7/48, A61K 38/47, C12N 15/86, C12N 15/62, A61K 48/00

(84) Designated Contracting States:
**AT BE CH CY DE DK ES FI FR GB GR IE IT LI LU MC NL PT SE**
Designated Extension States:
**AL LT LV MK**

(30) Priority: **28.07.1998 US 155203 P**
**07.12.1998 US 155254 P**

(62) Document number(s) of the earlier application(s) in accordance with Art. 76 EPC:
**99938848.1 / 1 100 905**

(71) Applicant: **INCYTE PHARMACEUTICALS, INC.**
**Palo Alto, CA 94304 (US)**

(72) Inventors:
- **Tang, Y., Tom**
  **San Jose, California 95118 (US)**
- **Lal, Preeti**
  **Santa Clara, CA 95056 (US)**
- **Corley, Neil C.**
  **Mountain View, CA 94040 (US)**
- **Guegler, Karl J.**
  **Menlo Park, CA 94025 (US)**
- **Patterson, Chandra**
  **Menlo Park, CA 94025 (US)**
- **Baughn, Mariah R.**
  **San Leandro, CA 94577 (US)**
- **Yue, Henry**
  **Sunnyvale, CA 94087 (US)**

(74) Representative: **Wright, Simon Mark**
**J.A. Kemp & Co.**
**14 South Square**
**Gray's Inn**
**London WC1R 5JJ (GB)**

Remarks:
This application was filed on 08 - 07 - 2004 as a divisional application to the application mentioned under INID code 62.

(54) **Human epidermal proteins HEPI-1 to HEPI-6**

(57) The invention provides human epidermal proteins (HEPI) and polynucleotides which identify and encode HEPI. The invention also provides expression vectors, host cells, antibodies, agonists and antagonists. The invention also provides methods for diagnosing, treating or preventing disorders associated with expression of HEPI.

EP 1 531 161 A2

SEQUENCE LISTING

<110> INCYTE PHARMACEUTICALS, INC.
TANG, Y. Tom
LAL, Preeti
CORLEY, Neil C.
GUEGLER, Karl J.
PATTERSON, Chandra
BAUGHN, Mariah R.
YUE, Henry

<120> HUMAN EPIDERMAL PROTEINS

<130> PF-0567 PCT

<140> To Be Assigned
<141> Herewith

<150> 09/123,531; unassigned; 09/206,817; unassigned
<151> 1998-07-28; 1998-07-28; 1998-12-07; 1998-12-07

<160> 16

<170> PERL Program

<210> 1
<211> 92
<212> PRT
<213> Homo sapiens

<220>
<221> misc_feature
<223> Incyte Clone No: 2024646

<400> 1
```
Met Ser Cys Gln Gln Asn Gln Gln Gln Cys Gln Pro Pro Pro Lys
  1               5                  10                  15
Cys Pro Ser Pro Lys Cys Pro Pro Lys Ser Pro Val Gln Cys Leu
                 20                  25                  30
Pro Pro Ala Ser Ser Gly Cys Ala Pro Ser Ser Gly Val Cys Gly
                 35                  40                  45
Pro Ser Ser Glu Gly Gly Cys Phe Leu Asn His His Arg Arg His
                 50                  55                  60
His Arg Cys Arg Arg Gln Arg Pro Asn Ser Cys Asp Arg Gly Ser
                 65                  70                  75
Gly Gln Gln Gly Gly Gly Ser Gly Cys Cys His Gly Ser Gly Gly
                 80                  85                  90
Cys Cys
```

<210> 2
<211> 118

<212> PRT
<213> Homo sapiens

<220>
<221> misc_feature
<223> Incyte Clone No: 3431776

<400> 2
```
Met Ser Cys Gln Gln Ser Gln Gln Gln Cys Gln Pro Pro Pro Lys
1               5                   10                  15
Cys Thr Pro Lys Cys Pro Pro Lys Cys Pro Thr Pro Lys Cys Pro
                20                  25                  30
Pro Lys Cys Pro Pro Lys Cys Pro Pro Val Ser Ser Cys Cys Ser
                35                  40                  45
Val Ser Ser Gly Gly Cys Cys Gly Ser Ser Ser Gly Gly Ser Cys
                50                  55                  60
Gly Ser Ser Ser Gly Gly Cys Cys Ser Ser Gly Gly Gly Gly Cys
                65                  70                  75
Cys Leu Ser His His Arg Arg Arg Arg Ser His Cys His Arg Pro
                80                  85                  90
Gln Ser Ser Gly Cys Cys Ser Gln Pro Ser Gly Gly Ser Ser Cys
                95                  100                 105
Cys Gly Gly Gly Ser Gly Gln His Ser Gly Gly Cys Cys
                110                 115
```

<210> 3
<211> 110
<212> PRT
<213> Homo sapiens

<220>
<221> misc_feature
<223> Incyte Clone No: 1798487

<400> 3
```
Met Ser Cys Gln Gln Asn Gln Gln Gln Cys Gln Pro Pro Pro Lys
1               5                   10                  15
Cys Pro Pro Lys Cys Thr Pro Lys Cys Pro Pro Lys Cys Pro Pro
                20                  25                  30
Lys Cys Pro Pro Gln Cys Pro Ala Pro Cys Phe Pro Ala Val Ser
                35                  40                  45
Ser Cys Cys Gly Pro Ser Ser Gly Ser Cys Cys Gly Pro Ser Ser
                50                  55                  60
Gly Gly Cys Cys Ser Ser Gly Ala Gly Gly Cys Ser Leu Ser His
                65                  70                  75
His Arg Pro Arg Leu Phe His Arg Arg Arg His Gln Ser Pro Asp
                80                  85                  90
Cys Cys Glu Ser Glu Pro Ser Gly Gly Ser Gly Cys Cys His Ser
                95                  100                 105
Ser Gly Gly Cys Cys
                110
```

<210> 4
<211> 128
<212> PRT
<213> Homo sapiens

<220>
<221> misc_feature
<223> Incyte Clone No: 1448744

<400> 4
```
Met Thr Gly Ser Cys Cys Gly Ser Thr Leu Ser Ser Leu Ser Tyr
 1               5                  10                  15
Gly Gly Gly Cys Cys Gln Pro Cys Cys Cys Arg Tyr Pro Cys Cys
                20                  25                  30
Cys Arg Pro Val Thr Cys Gln Thr Thr Val Cys Arg Pro Val Thr
                35                  40                  45
Cys Val Pro Arg Cys Thr Arg Pro Ile Cys Glu Pro Cys Cys Arg
                50                  55                  60
Pro Val Cys Cys Asp Pro Cys Ser Leu Gln Glu Gly Cys Cys Arg
                65                  70                  75
Pro Ile Thr Cys Cys Pro Ser Ser Cys Thr Ala Val Val Cys Arg
                80                  85                  90
Pro Cys Cys Trp Ala Thr Thr Cys Cys Gln Pro Val Ser Val Gln
                95                  100                 105
Ser Pro Cys Cys Arg Pro Pro Cys Gly Gln Pro Thr Pro Cys Ser
                110                 115                 120
Thr Thr Cys Arg Thr Ser Ser Cys
                125
```

<210> 5
<211> 342
<212> PRT
<213> Homo sapiens

<220>
<221> misc_feature
<223> Incyte Clone No: 2737275

<400> 5
```
Met Asn Asn Pro Ile Pro Ser Asn Leu Lys Ser Glu Ala Lys Lys
 1               5                  10                  15
Ala Ala Lys Ile Leu Arg Glu Phe Thr Glu Ile Thr Ser Arg Asn
                20                  25                  30
Gly Pro Asp Lys Ile Ile Pro Ala His Val Ile Ala Lys Ala Lys
                35                  40                  45
Gly Leu Ala Ile Leu Ser Val Ile Lys Ala Gly Phe Leu Val Thr
                50                  55                  60
Ala Arg Gly Gly Ser Gly Ile Val Val Ala Arg Leu Pro Asp Gly
                65                  70                  75
Lys Trp Ser Ala Pro Ser Ala Ile Gly Ile Ala Gly Leu Gly Gly
```

80 85 90
Gly Phe Glu Ile Gly Ile Glu Val Ser Asp Leu Val Ile Ile Leu
95 100 105
Asn Tyr Asp Arg Ala Val Glu Ala Phe Ala Lys Gly Gly Asn Leu
110 115 120
Thr Leu Gly Gly Asn Leu Thr Val Ala Val Gly Pro Leu Gly Arg
125 130 135
Asn Leu Glu Gly Asn Val Ala Leu Arg Ser Ser Ala Ala Val Phe
140 145 150
Thr Tyr Cys Lys Ser Arg Gly Leu Phe Ala Gly Val Ser Leu Glu
155 160 165
Gly Ser Cys Leu Ile Glu Arg Lys Glu Thr Asn Arg Lys Phe Tyr
170 175 180
Cys Gln Asp Ile Arg Ala Tyr Asp Ile Leu Phe Gly Asp Thr Pro
185 190 195
Arg Pro Ala Gln Ala Glu Asp Leu Tyr Glu Ile Leu Asp Ser Phe
200 205 210
Thr Glu Lys Tyr Glu Asn Glu Gly Gln Arg Ile Asn Ala Arg Lys
215 220 225
Ala Ala Arg Glu Gln Arg Lys Ser Ser Ala Lys Glu Leu Pro Pro
230 235 240
Lys Pro Leu Ser Arg Pro Gln Gln Ser Ser Ala Pro Val Gln Leu
245 250 255
Asn Ser Gly Ser Gln Ser Asn Arg Asn Glu Tyr Lys Leu Tyr Pro
260 265 270
Gly Leu Ser Ser Tyr His Glu Arg Val Gly Asn Leu Asn Gln Pro
275 280 285
Ile Glu Val Thr Ala Leu Tyr Ser Phe Glu Gly Gln Gln Pro Gly
290 295 300
Asp Leu Asn Phe Gln Ala Gly Asp Arg Ile Thr Val Ile Ser Lys
305 310 315
Thr Asp Ser His Phe Asp Trp Trp Glu Gly Lys Leu Arg Gly Gln
320 325 330
Thr Gly Ile Phe Pro Ala Asn Tyr Val Thr Met Asn
335 340

<210> 6
<211> 529
<212> PRT
<213> Homo sapiens

<220>
<221> misc_feature
<223> Incyte Clone No: 3325323

<400> 6
Met Gly Ser Ser Arg Ala Pro Trp Met Gly Arg Val Gly Gly His
1 5 10 15
Gly Met Leu Ala Leu Leu Leu Ala Gly Leu Leu Leu Pro Gly Thr
20 25 30
Leu Ala Lys Ser Ile Gly Thr Phe Ser Asp Pro Cys Lys Asp Pro
35 40 45

Thr Arg Ile Thr Ser Pro Asn Asp Pro Cys Leu Thr Gly Lys Gly
50 55 60
Asp Ser Ser Gly Phe Ser Ser Tyr Ser Gly Ser Ser Ser Ser Gly
65 70 75
Ser Ser Ile Ser Ser Ala Arg Ser Ser Gly Gly Gly Ser Ser Gly
80 85 90
Ser Ser Ser Gly Ser Ser Ile Ala Gln Gly Gly Ser Ala Gly Ser
95 100 105
Phe Lys Pro Gly Thr Gly Tyr Ser Gln Val Ser Tyr Ser Ser Gly
110 115 120
Ser Gly Ser Ser Leu Gln Gly Ala Ser Gly Ser Ser Gln Leu Gly
125 130 135
Ser Ser Ser Ser His Ser Gly Ser Ser Gly Ser His Ser Gly Ser
140 145 150
Ser Ser Ser His Ser Ser Ser Ser Ser Ser Phe Gln Phe Ser Ser
155 160 165
Ser Ser Phe Gln Val Gly Asn Gly Ser Ala Leu Pro Thr Asn Asp
170 175 180
Asn Ser Tyr Arg Gly Ile Leu Asn Pro Ser Gln Pro Gly Gln Ser
185 190 195
Ser Ser Ser Ser Gln Thr Phe Gly Val Ser Ser Ser Gly Gln Ser
200 205 210
Val Ser Ser Asn Gln Arg Pro Cys Ser Ser Asp Ile Pro Asp Ser
215 220 225
Pro Cys Ser Gly Gly Pro Ile Val Ser His Ser Gly Pro Tyr Ile
230 235 240
Pro Ser Ser His Ser Val Ser Gly Gly Gln Arg Pro Val Val Val
245 250 255
Val Val Asp Gln His Gly Ser Gly Ala Pro Gly Val Val Gln Gly
260 265 270
Pro Pro Cys Ser Asn Gly Gly Leu Pro Gly Lys Pro Cys Pro Pro
275 280 285
Ile Thr Ser Val Asp Lys Ser Tyr Gly Gly Tyr Glu Val Val Gly
290 295 300
Gly Ser Ser Asp Ser Tyr Leu Val Pro Gly Met Thr Tyr Ser Lys
305 310 315
Gly Lys Ile Tyr Pro Val Gly Tyr Phe Thr Lys Glu Asn Pro Val
320 325 330
Lys Gly Ser Pro Gly Val Pro Ser Phe Ala Ala Gly Pro Pro Ile
335 340 345
Ser Glu Gly Lys Tyr Phe Ser Ser Asn Pro Ile Ile Pro Ser Gln
350 355 360
Ser Ala Ala Ser Ser Ala Ile Ala Phe Gln Pro Val Gly Thr Gly
365 370 375
Gly Val Gln Leu Cys Gly Gly Gly Ser Thr Gly Ser Lys Gly Pro
380 385 390
Cys Ser Pro Ser Ser Ser Arg Val Pro Ser Ser Ser Ser Ile Ser
395 400 405
Ser Ser Ser Gly Leu Pro Tyr His Pro Cys Gly Ser Ala Ser Gln
410 415 420
Ser Pro Cys Ser Pro Pro Gly Thr Gly Ser Phe Ser Ser Ser Ser
425 430 435
Ser Ser Gln Ser Ser Gly Lys Ile Ile Leu Gln Pro Cys Gly Ser
440 445 450
Lys Ser Ser Ser Ser Gly His Pro Cys Met Ser Val Ser Ser Leu

```
                    455                     460                     465
Thr Leu Thr Gly Gly Pro Asp Gly Ser Pro His Pro Asp Pro Ser
                    470                     475                     480
Ala Gly Ala Lys Pro Cys Gly Ser Ser Ser Ala Gly Lys Ile Pro
                    485                     490                     495
Cys Arg Ser Ile Arg Asp Ile Leu Ala Gln Val Lys Pro Leu Gly
                    500                     505                     510
Pro Gln Leu Ala Asp Pro Glu Val Phe Leu Pro Gln Gly Glu Leu
                    515                     520                     525
Leu Asp Ser Pro
```

<210> 7
<211> 603
<212> DNA
<213> Homo sapiens

<220>
<221> misc_feature
<223> Incyte Clone No: 2024646

<400> 7

```
caggaacagc cttctcctgc ctcctctgca cctggacaac tcaactcctg ccaagatgtc  60
ctgccagcag aaccagcagc agtgccaacc cccacccaag tgtccctcac ccaagtgtcc 120
cccaaagagc ccagtacagt gtctgcctcc agcttcctct ggctgtgccc caagctctgg 180
ggtctgtggc cctagctccg agggcggctg cttcctgaac caccacaggc gccaccaccg 240
atgccggcgc cagaggccca actcctgtga caggggcagt ggtcagcaag gcgggggctc 300
tggctgctgc cacggttctg gggctgctg ctgatccaga tcctgatgct gagacaagcg 360
atctttggag gaaacaagaa tcccaagagg ccaagaacag ccccatctga cgcatgcctt 420
cccatatacc ctcttctgac tttcacaggc tgagctggag gttttcctgt gggggatctg 480
agctctcccc agaaggcact tcttgtttta tgtacaggat gtcatatgtc cccctacccc 540
tgtacctgcc aaggattggc agtgcttgtg cccaacctcg taaaaaagat aaagttccgt 600
tgc                                                               603
```

<210> 8
<211> 697
<212> DNA
<213> Homo sapiens

<220>
<221> misc_feature
<223> Incyte Clone No: 3431776

<400> 8

```
ccgaggtgct gaaggaccct gtgctgcctg tgaccccgct cctgaatccg ccaccaagat  60
gtcctgccag cagagccagc agcagtgcca gccccctccc aagtgcaccc ccaagtgccc 120
tcccaagtgc cccaccccaa agtgtccccc aaagtgtccc cctaagtgcc ctcctgtctc 180
ttcctgctgc agtgtcagct ccggaggctg ctgtggctcc agctctgggg gcagctgtgg 240
ctccagctct gggggatgct gcagttctgg gggaggtggc tgctgcctga gccaccacag 300
gcgccgtagg tcccactgcc acagacccca gagctctggc tgctgcagcc agccctcggg 360
gggctccagc tgctgtggcg gggggagtgg ccagcactct ggaggctgct gctgaagtgg 420
accctgagcc tagaagagca gaatccagga ccgcaaactg ccaaggacat cccccttctc 480
```

ctactaggcc tgcctgagag gctcacaggt ccaagggaaa gctctgaact tgccaagagc 540
atatcttttc cctggagtcc agaaactcag atcctctcct ggatctccat tcactggcct 600
tggacctcac ctttgtggct accctcccac gctctgtcta agcccctagc ttactcaatg 660
tcatttgcag cgtgcatctg ctgattaacc gacgcaa 697

<210> 9
<211> 528
<212> DNA
<213> Homo sapiens

<220>
<221> misc_feature
<223> Incyte Clone No: 1798487

<400> 9
atgtcttgcc agcaaaacca gcagcagtgc cagccccctc ccaagtgtcc tcccaagtgt 60
accccaaaat gtccacctaa gtgtcccccc aaatgcccac cacagtgccc agctccatgt 120
ttccctgcag tctcttcttg ctgtggtccc agctctggga gctgctgtgg tcccagctct 180
gggggctgct gcagctctgg ggctggtggc tgctccctga gccaccacag gccccgtctc 240
ttccaccggc gccggcacca gagccccgac tgctgtgaga gtgaaccttc tgggggctct 300
ggctgctgcc acagctctgg gggctgctgc tgacctgggc tacagaagag ctcttgggac 360
tgaatggcca agaacctgct acggcctgat ggatactctt tccacttcct ctcattccat 420
tcattggttg gcagagacca caaagactca tggggctttc ctggaagaac ttcgtgcttg 480
aatgtaacac cccaattgaa agtcttcttt tcctccgttt acctcatg 528

<210> 10
<211> 493
<212> DNA
<213> Homo sapiens

<220>
<221> misc_feature
<223> Incyte Clone No: 1448744

<400> 10
caacagccca acccacacca gcctcagaca ccaccatgac cggctcctgc tgcggctcca 60
ccttgtcctc cctgagctac gggggaggct gctgccagcc ctgctgctgc cgctacccct 120
gctgctgccg ccccgtgacc tgccagacca ccgtgtgccg ccccgtgacc tgcgtgcccc 180
gctgcacgcg ccccatctgc gagccctgct gccgcccggt gtgctgcgac ccctgctccc 240
tgcaggaagg ctgctgccgc cccatcacct gctgcccctc gtcgtgcacg gctgtggtgt 300
gcaggccctg ctgctgggcc accacctgct gccagcctgt gtctgtgcag tccccctgct 360
gccggcctcc ctgcggccag ccgacccctt gcagcaccac ctgcaggacc tcctcctgct 420
gagaccccac ctctcctctc atcgcacgaa acattcccag gtgcacagaa tcttgtgcag 480
actcttctac ccc 493

<210> 11
<211> 1332
<212> DNA
<213> Homo sapiens

<220>
<221> misc_feature

<223> Incyte Clone No: 2737275

<400> 11
gttctagatc gcgagcggcc gcccgcgatc tagaactagt ctcgggacgg ggcgccgcgg 60
ccgggcgggc agcatgaata accctatacc ttccaatttg aaatcagaag caaaaaaggc 120
tgccaaaata ttaagagaat tcacagaaat aacttccaga aatggacctg ataagatcat 180
tcctgctcac gtaattgcga aggctaaagg ccttgcaatt ctgtctgtga tcaaagccgg 240
gttcctggtg actgccagag gaggcagcgg gattgtagtg gcgcgccttc cagatggaaa 300
atggtctgca ccctcagcca ttgggatagc tggccttggt ggaggatttg aaataggaat 360
tgaggtatca gacttggtga taattctgaa ttatgaccgt gctgtagaag cttttgcaaa 420
aggcggaaat ctgaccctcg gagggaactt gactgtggcg gttgggccct tgggaaggaa 480
cttggaagga aacgtggccc tgagaagctc cgctgccgtc ttcacgtact gcaagtcaag 540
gggactcttt gcaggcgtgt ctttagaagg gagctgtttg attgaaagga aagaaactaa 600
tagaaaattt tattgtcaag atatccgagc ttatgacatt ttatttggag atacaccgcg 660
gcctgctcaa gccgaagatc tttatgaaat tcttgattcc tttactgaaa agtatgaaaa 720
tgaaggacaa cgaatcaatg caagaaaagc agcaagggag cagaggaagt cttctgctaa 780
agaattacct ccaaagccat tgtcaagacc acagcagtca tctgcaccag tccagctgaa 840
ctctggctct caaagtaaca gaaatgaata taagctctat cctggacttt ccagctatca 900
tgagagagtt ggcaatttga atcaacccat agaagtgaca gcgctgtatt catttgaagg 960
acagcagcct ggggatttga attttcaagc tggagacaga atcacagtta tatcaaaaac 1020
agattcacat tttgattggt gggaaggaaa acttcgaggt caaactggca tttttccagc 1080
caactacgta accatgaatt aaagcgtata ctattttctt ctttgagaat tacaaaaaaa 1140
ttatttctac actgacagga tttactagtt aagcaatgtt taatataaat tttaaaaaac 1200
ttctgttcta caaaatttcc attccgtatg taaaagattt tgtttttcta tataaaaaga 1260
gctgactgac atatctttaa atactttgta ctaactttat cacacttact gtgtcataga 1320
atatcataca gt 1332

<210> 12
<211> 2284
<212> DNA
<213> Homo sapiens

<220>
<221> misc_feature
<223> Incyte Clone No: 3325323

<400> 12
caggtgtcct cgagctgcca tcagtcagga ggccgtgcag tccgagatgg gctcgtctcg 60
ggcaccctgg atggggcgtg tgggtgggca cgggatgttg gcactgctgc tggctggtct 120
cctcctgcca gggaccttgg ctaagagcat tggcaccttc tcagacccct gtaaggaccc 180
cacgcgtatc acctccccta acgacccctg cctcactggg aagggtgact ccagcggctt 240
cagtagctac agtggctcca gcagttctgg cagctccatt tccagtgcca gaagctctgg 300
tggtggctcc agtggtagct ccagcggatc cagcattgcc cagggtggtt ctgcaggatc 360
ttttaagcca ggaacggggt attcccaggt cagctactcc tccggatctg gctctagtct 420
acaaggtgca tccggttcct cccagctggg gagcagcagc tctcactcgg gaagcagcgg 480
ctctcactcg ggaagcagca gctctcattc gagcagcagc agcagctttc agttcagcag 540
cagcagcttc caagtaggga atggctctgc tctgccaacc aatgacaact cttaccgcgg 600
aatactaaac ccttcccagc ctggacaaag ctcttcctct tcccagacct ttggggtatc 660
cagcagtggc caaagcgtca gctccaacca gcgtccctgt agttcggaca tccccgactc 720
tccctgcagt ggagggccca tcgtctcgca ctccggcccc tacatcccca gctcccactc 780
tgtgtcaggg ggtcagaggc ctgtggtggt ggtggtggac cagcacggtt ctggtgcccc 840
tggagtggtt caaggtcccc cctgtagcaa tggtggcctt ccaggcaagc cctgtccccc 900
aatcacctct gtagacaaat cctatggtgg ctacgaggtg gtgggtggct cctctgacag 960
ttatctggtt ccaggcatga cctacagtaa gggtaaaatc taccctgtgg gctacttcac 1020

```
caaagagaac cctgtgaaag gctctccagg ggtcccttcc tttgcagctg ggccccccat 1080
ctctgagggc aaatacttct ccagcaaccc catcatcccc agccagtcgg cagcttcctc 1140
ggccattgca ttccagccag tggggactgg tggggtccag ctctgtggag gcggctccac 1200
gggctccaag ggaccctgct ctccctccag ttctcgagtc cccagcagtt ctagcatttc 1260
cagcagctcc ggtttaccct accatccctg cggcagtgct tcccagagcc cctgctcccc 1320
accaggcacc ggctccttca gcagcagctc cagttcccaa tccagtggca aaatcatcct 1380
tcagccttgc ggcagcaagt ccagctcttc tggtcaccct tgcatgtctg tctcctcctt 1440
gacactgact gggggccccg atggctctcc ccatcctgat ccctccgctg gtgccaagcc 1500
ctgtggctcc agcagtgctg gaaagatccc ctgccgctcc atccgggata tcctagccca 1560
agtgaagcct ctggggcccc agctagctga ccctgaagtt ttcctacccc aaggagagtt 1620
actcgacagt ccataagaag tcaactgttg tgtgtgtgca tgccttgggc acaaacaagc 1680
acatacacta tatcccatat gggagaagtc cagtgcccag gcatagggtt agctcagttt 1740
ccctccttcc caaaagagtg gttctgcttt ctccactacc ccaaggttgc agactctctc 1800
ttatcacccc ttcctccttc ctcttctcaa aatggtagat tcaaagctcc tctcttgatt 1860
ctctcctact gtttaaattc ccattccacc acagtgcccc tcagccagat caccacccct 1920
tacaattccc tctactgtgt ggaaatggtc cattgagtaa cacccccatc agcttctcaa 1980
ctgggaaacc cctgaaatgc tctcagagca cctctgacgc ctgaagaagt tataccttcc 2040
tcttcccctt taccaaataa agcaaagtca aaccatcatc tggaaacagt ggccactttt 2100
cactgacctt tcttcgacat ctagtcaacc cacccaatat gccactgggc tctcgctccc 2160
aattccaccc caccctccat tacagagctc accacgccct cctagatcac cgtccccaac 2220
acacccattg cctctcaagg cccttatctc agccccttcc tgtgggggga tcctctagag 2280
tcga                                                              2284
```

<210> 13
<211> 110
<212> PRT
<213> Homo sapiens

<220>
<221> misc_feature
<223> GenBank ID No: g2589188

<400> 13

```
Met Ser Cys Gln Gln Asn Gln Gln Gln Cys Gln Pro Pro Pro Lys
  1               5                  10                  15
Cys Pro Pro Lys Cys Thr Pro Lys Cys Pro Pro Lys Cys Pro Pro
                 20                  25                  30
Lys Cys Leu Pro Gln Cys Pro Ala Pro Cys Ser Pro Ala Val Ser
                 35                  40                  45
Ser Cys Cys Gly Pro Ile Ser Gly Gly Cys Cys Gly Pro Ser Ser
                 50                  55                  60
Gly Gly Cys Cys Asn Ser Gly Ala Gly Gly Cys Cys Leu Ser His
                 65                  70                  75
His Arg Pro Arg Leu Phe His Arg Arg Arg His Gln Ser Pro Asp
                 80                  85                  90
Cys Cys Glu Ser Glu Pro Ser Gly Gly Ser Gly Cys Cys His Ser
                 95                 100                 105
Ser Gly Gly Cys Cys
                110
```

<210> 14

<211> 131
<212> PRT
<213> Ovis aries

<220>
<221> misc_feature
<223> GenBank ID No: g71384

<400> 14
Thr Gly Ser Cys Cys Gly Pro Thr Phe Ser Ser Leu Ser Cys Gly
1 5 10 15
Gly Gly Cys Leu Gln Pro Arg Tyr Tyr Arg Asp Pro Cys Cys Cys
20 25 30
Arg Pro Val Ser Cys Gln Thr Val Ser Arg Pro Val Thr Phe Val
35 40 45
Pro Arg Cys Thr Arg Pro Ile Cys Glu Pro Cys Arg Arg Pro Val
50 55 60
Cys Cys Asp Pro Cys Ser Leu Gln Glu Gly Cys Cys Arg Pro Ile
65 70 75
Thr Cys Cys Pro Thr Ser Cys Gln Ala Val Val Cys Arg Pro Cys
80 85 90
Cys Trp Ala Thr Thr Cys Cys Gln Pro Val Ser Val Gln Cys Pro
95 100 105
Cys Cys Arg Pro Thr Ser Cys Gln Pro Ala Pro Cys Ser Arg Thr
110 115 120
Thr Cys Arg Thr Phe Arg Thr Ser Pro Cys Cys
125 130

<210> 15
<211> 340
<212> PRT
<213> Mus musculus

<220>
<221> misc_feature
<223> GenBank ID No: g1944389

<400> 15
Met Asn Asn Pro Ile Pro Ser Asn Leu Lys Ser Glu Ala Lys Lys
1 5 10 15
Ala Ala Lys Ile Leu Arg Glu Phe Thr Glu Ile Thr Ser Arg Asn
20 25 30
Gly Pro Asp Lys Ile Ile Pro Ala His Val Ile Ala Lys Ala Lys
35 40 45
Gly Leu Ala Val Leu Ser Val Ile Lys Ala Gly Phe Leu Val Thr
50 55 60
Ala Arg Gly Gly Ser Gly Ile Val Leu Ala Arg Leu Pro Asp Gly
65 70 75
Lys Trp Ser Ala Pro Ser Ala Ile Gly Ile Ala Gly Leu Gly Gly
80 85 90
Gly Phe Glu Ile Gly Ile Glu Val Ser Asp Leu Val Ile Ile Leu
95 100 105

Asn Tyr Asp Arg Ala Val Glu Ala Phe Ala Lys Gly Gly Asn Leu
110 115 120
Thr Leu Gly Gly Asn Phe Thr Val Ala Val Gly Pro Leu Gly Arg
125 130 135
Asn Leu Glu Gly Asn Val Ser Leu Arg Ser Ser Ala Ala Val Phe
140 145 150
Thr Tyr Cys Lys Ser Arg Gly Leu Phe Ala Gly Ile Ser Leu Glu
155 160 165
Gly Ser Cys Leu Ile Glu Arg Lys Glu Thr Asn Arg Lys Phe Tyr
170 175 180
Cys Gln Asp Ile Arg Ala Tyr Asp Ile Leu Phe Gly Asp Val Pro
185 190 195
Gln Pro Ala Gln Ala Glu Asp Leu Tyr Glu Ile Leu Asn Ser Phe
200 205 210
Thr Glu Lys Tyr Glu Thr Glu Gly Gln Arg Ile Asn Leu Lys Lys
215 220 225
Val Ala Arg Glu Gln Arg Lys Ala Lys Glu Leu Pro Pro Lys Pro
230 235 240
Ser Ser Arg Pro Gln Pro Ala His Pro Pro Val Gln Leu Asn Ala
245 250 255
Gly Ser Gln Gly Asn Arg Asn Glu Tyr Lys Leu Tyr Pro Glu Leu
260 265 270
Ser Ser Tyr His Glu Lys Thr Gly Asn Leu Asn Gln Pro Ile Glu
275 280 285
Val Thr Ala Leu Tyr Ser Phe Glu Gly Gln Gln Pro Gly Asp Leu
290 295 300
Asn Phe Gln Ala Gly Asp Arg Ile Ile Val Ile Ser Lys Thr Asp
305 310 315
Ser Asn Phe Asp Trp Trp Glu Gly Lys Leu Arg Gly Gln Thr Gly
320 325 330
Ile Phe Pro Ala Asn Tyr Val Thr Met Asn
335 340

<210> 16
<211> 486
<212> PRT
<213> Homo sapiens

<220>
<221> misc_feature
<223> GenBank ID No: g414810

<400> 16
Met Leu Ala Leu Leu Leu Ala Gly Leu Leu Leu Pro Gly Thr Leu
1 5 10 15
Ala Lys Ser Ile Gly Thr Phe Ser Asp Pro Cys Lys Asp Pro Thr
20 25 30
Arg Ile Thr Ser Pro Asn Asp Pro Cys Leu Thr Gly Lys Gly Asp
35 40 45
Ser Ser Gly Phe Ser Ser Tyr Ser Gly Ser Ser Ser Ser Gly Ser
50 55 60
Ser Ile Ser Ser Ala Arg Ser Ser Gly Gly Gly Ser Ser Gly Ser
65 70 75
Ser Ser Gly Ser Ser Ile Ala Gln Gly Gly Ser Ala Gly Ser Phe
80 85 90

```
Lys Pro Gly Thr Gly Tyr Ser Gln Val Ser Tyr Ser Ser Gly Ser
                95                  100                 105
Gly Ser Ser Leu Gln Gly Ala Ser Gly Ser Ser Gln Leu Gly Ser
                110                 115                 120
Ser Ser Ser His Ser Gly Ser Ser Gly Ser His Ser Gly Ser Ser
                125                 130                 135
Ser Ser His Ser Ser Ser Ser Ser Ser Phe Gln Phe Ser Ser Ser
                140                 145                 150
Ser Phe Gln Val Gly Asn Gly Ser Ala Leu Pro Thr Asn Asp Asn
                155                 160                 165
Ser Tyr Arg Gly Ile Leu Asn Pro Ser Gln Pro Gly Gln Ser Ser
                170                 175                 180
Ser Ser Ser Gln Thr Ser Gly Val Ser Ser Ser Gly Gln Ser Val
                185                 190                 195
Ser Ser Asn Gln Arg Pro Cys Ser Ser Asp Ile Pro Asp Ser Pro
                200                 205                 210
Cys Ser Gly Gly Pro Ile Val Ser His Ser Gly Pro Tyr Ile Pro
                215                 220                 225
Ser Ser His Ser Val Ser Gly Gly Gln Arg Pro Val Val Val Val
                230                 235                 240
Val Asp Gln His Gly Ser Gly Ala Pro Gly Val Val Gln Gly Pro
                245                 250                 255
Pro Cys Ser Asn Gly Gly Leu Pro Gly Lys Pro Cys Pro Pro Ile
                260                 265                 270
Thr Ser Val Asp Lys Ser Tyr Gly Gly Tyr Glu Val Val Gly Gly
                275                 280                 285
Ser Ser Asp Ser Tyr Leu Val Pro Gly Met Thr Tyr Ser Lys Gly
                290                 295                 300
Lys Ile Tyr Pro Val Gly Tyr Phe Thr Lys Glu Asn Pro Val Lys
                305                 310                 315
Gly Ser Pro Gly Val Pro Ser Phe Ala Ala Gly Pro Pro Ile Ser
                320                 325                 330
Glu Gly Lys Tyr Phe Ser Ser Asn Pro Ile Ile Pro Ser Gln Ser
                335                 340                 345
Ala Ala Ser Ser Ala Ile Ala Phe Gln Pro Val Gly Thr Gly Gly
                350                 355                 360
Val Gln Leu Cys Gly Gly Gly Ser Thr Gly Ser Lys Gly Pro Cys
                365                 370                 375
Ser Pro Ser Ser Ser Arg Val Pro Ser Ser Ser Ser Ile Ser Ser
                380                 385                 390
Ser Ser Gly Ser Pro Tyr His Pro Cys Gly Ser Ala Ser Gln Ser
                395                 400                 405
Pro Cys Ser Pro Pro Gly Thr Gly Ser Phe Ser Ser Ser Ser Ser
                410                 415                 420
Ser Gln Ser Ser Gly Lys Ile Ile Leu Gln Pro Cys Gly Ser Lys
                425                 430                 435
Ser Ser Ser Ser Gly His Pro Cys Met Ser Val Ser Ser Leu Thr
                440                 445                 450
Leu Thr Gly Gly Pro Asp Gly Ser Pro His Pro Asp Pro Ser Ala
                455                 460                 465
Gly Ala Lys Pro Cys Gly Ser Ser Ser Ala Gly Lys Ile Pro Cys
                470                 475                 480
Arg Ser Ile Arg Ile Ser
                485
```

## Description

### TECHNICAL FIELD

This invention relates to nucleic acid and amino acid sequences of human epidermal proteins and to the use of these sequences in the diagnosis, treatment, and prevention of epithelial, cell proliferative, and autoimmune/inflammatory disorders.

### BACKGROUND OF THE INVENTION

Skin protects the body against desiccation and chemical, biological, and physical injury. Skin also senses environmental stimuli through tactile sense organs and plays an important role in water balance and thermoregulation. Moving from outermost to innermost, the three major skin layers are the epidermis, the dermis, and the hypodermis. The properties of the epidermis largely determine the specific functions of the skin.

The epidermis is a stratified squamous epithelium composed of multiple layers of cells called keratinocytes. On the outermost part of the epidermis, the stratum corneum, are layers of non-living cells derived from keratinocytes. These dead cells, termed corneocytes, are filled with keratin protein filaments (intermediate filaments). The dead cells are constantly shed in a process called desquamation. Keratinocytes in the basal layer of the epidermis (adjoining the dermis) divide continuously to replace the dead cells lost to desquamation. These basal keratinocytes are the only epidermal cells that undergo cell division.

Newly formed keratinocytes move toward the skin surface as they are displaced by the cell proliferation occurring in the basal layer. As the keratinocytes move toward the skin surface, they differentiate, grow larger, and accumulate keratin filaments in their cytoplasm. Various keratins are synthesized as the cells progress through their differentiation program. Other proteins produced in a regulated manner in differentiating keratinocytes include the keratin assembly protein filaggrin and envelope proteins such as involucrin and loricrin that reside on the inner cell membrane of the keratinocyte. Membrane-coating granules that later release lipids and lipoproteins into the intercellular spaces are also formed during the differentiation process. The permeability of the cells to calcium ions increases during differentiation. Calcium ions activate an enzyme that crosslinks the envelope proteins to form a very tough layer beneath the cell membrane. Finally, lysosomes in the cells release lytic enzymes that terminate all metabolic activity, leaving dead, fully keratinized cells that become part of the stratum corneum. (Fawcett, D.W. (1994) Bloom and Fawcett, A Textbook of Histology, Chapman and Hall, New York, NY pp. 525-531.)

Comeodesmosomes are structures that are proposed to adhere adjacent corneocytes to one another in the stratum corneum. Corneodesmosin, the product of the S gene, forms part of the comeodesmosome, and corneodesmosin's proteolysis may be associated with desquamation. Corneodesmosin is expressed in cornified cell envelopes and in such cornified squamous epithelia as epidermis, hard palate, epithelium, and inner root sheath of the hair follicle. In skin, corneodesmosin is expressed in keratinocytes that are entering the terminal phase of their differentiation pathway. Corneodesmosin, an N-glycosylated protein, is rich in serine, glycine, and proline. The S gene is located within the Class I region of the HLA complex, and alleles of HLA-C are associated with psoriasis vulgaris, a skin disease. (See Zhou, Y. and Chaplin, D.D. (1993) Proc. Natl. Acad. Sci. USA 90:9470-9474; Simon, M. et al. (1997) J. Biol. Chem. 272:31770-31776.)

The epidermal differentiation complex (EDC) is a region of chromosome 1q21 that contains over twenty genes involved in terminal differentiation of the epidermis. Identified proteins encoded in the EDC include the comified envelope proteins, loricrin and involucrin (IVL); at least eleven small proline-rich region (SPRR) proteins; the intermediate filament-associated proteins, profilaggrin and trichohyalin, and several S100A calcium-binding proteins. The SPRR proteins are 8-10 kDa in molecular mass, rich in proline, glutamine, and cysteine, and contain similar repeating sequence elements. The SPRR proteins may be structural proteins with a strong secondary structure or metal-binding proteins such as metallothioneins. The involucrin and loricrin genes may have evolved from the SPRR genes. (Online Mendelian Inheritance In Man (OMIM) #601588 Epidermal Differentiation Complex; Mischke, D. et al. (1996) J. Invest. Dermatol. 106:989-992; Kartasova, T. and van de Putte, P. (1988) Mol. Cell. Biol. 8:2195-2203; and Zhao, X.P. and Elder, J.T. (1997) Genomics 45:250-258.)

SPRR genes are preferentially expressed in differentiating keratinocytes and are also expressed at high levels in stratified epithelia of the upper digestive tract and in tissues characterized by regenerative epidermal maturation. (Zhao, supra.) Expression of SPRR genes is increased in human epidermal keratinocytes after both UV irradiation and treatment with 4-nitroquinoline 1-oxide or 12-O-tetradecanoylphorbol 13-acetate. (Kartasova, supra.) Recently a novel skin-specific gene, xp5, was identified in the SPRR/IVL region of the EDC. The xp5 gene is expressed at high levels in normal and psoriatic skin, but not in cultured keratinocytes or in a variety of cell lines and human tissues. (Zhao, supra.)

Hair is a keratinized tissue formed within the hair follicle as a differentiation product of the epidermis. Hair cells contain bundles of keratin filaments (intermediate filaments) with an interstitial matrix composed of cysteine-rich and glycine/tyrosine-rich keratin-associated proteins. A large family of cysteine-rich keratin-associated proteins, also known as high-sulfur and ultra high-sulfur proteins, has been characterized in mammals. These proteins contain from 12 to 40 mol % cysteine. The cysteines may crosslink the intermediate filaments and thus stabilize the structure of hair cells. Multiple motifs containing two cysteines, a charged amino acid, and a proline are found in the cysteine-rich keratin-associated proteins. The cysteine-rich keratin-associated proteins are expressed in the hair follicle and in the skin during the growth phase of hair. (See Wood, L. et al. (1990) J. Biol. Chem. 265:21375-21380; Powell, B.C. et al. (1995) Differentiation 58:227-232.)

Several disorders are associated with epidermal differentiation. Psoriasis, a chronic inflammatory dermatosis which affects approximately 2% of the population, is characterized by epidermal hyperproliferation and inflammation. In some cases psoriasis is associated with arthritis. Psoriasis has a genetic component; psoriasis susceptibility loci have been linked to the HLA region and to chromosome 17q. Suggestive linkages to chromosome 16q and chromosome 20p regions have also been noted. Psoriasis is aggravated by streptococcal infection, and a protein of group A beta-hemolytic streptococcus cross-reacts with certain HLA-A antigens. Psoriasis is much more common in patients with Crohn's disease, an inflammatory disorder of the gastrointestinal tract, than in controls; an immunomodulatory locus capable of influencing both psoriasis and Crohn's disease may reside in chromosome 16q. Other skin disorders associated with hyperproliferation of the epidermis include inflammatory allergic diseases, chronic wounds, and skin cancer. (OMIM * 177900 Psoriasis Susceptibility 1; Nair, R.P. et al. (1997) Hum. Mol. Genet. 6:1349-1356; and Gniadecki, R. (1998) Gen. Pharmac. 30:619-622.)

Proteins of the EDC also have disease associations. The calcium-binding proteins S100A7, S100A8, and S100A9 are upregulated in psoriatic epidermis and in primary keratinocytes undergoing aberrant differentiation. Other EDC-associated diseases include Vohwinkel keratoderma, a mutilating hyperkeratosis caused by a molecular defect in loricrin, and certain disorders of keratinization (icthyoses), in which there is decreased expression of profilaggrin. (OMIM #601588, supra.)

The discovery of new human epidermal proteins and the polynucleotides encoding them satisfies a need in the art by providing new compositions which are useful in the diagnosis, prevention, and treatment of epithelial, cell proliferative, and autoimmune/inflammatory disorders.

## SUMMARY OF THE INVENTION

The invention features substantially purified polypeptides, human epidermal proteins, referred to collectively as "HEPI" and individually as "HEPI-1", "HEPI-2", "HEPI-3", "HEPI-4", "HEPI-5", and "HEPI-6". In one aspect, the invention provides a substantially purified polypeptide comprising an amino acid sequence selected from the group consisting of SEQ ID NO: 1-6 and fragments thereof.

The invention further provides a substantially purified variant having at least 90% amino acid identity to at least one of the amino acid sequences selected from the group consisting of SEQ ID NO:1-6 and fragments thereof. The invention also provides an isolated and purified polynucleotide encoding the polypeptide comprising an amino acid sequence selected from the group consisting of SEQ ID NO:1-6 and fragments thereof. The invention also includes an isolated and purified polynucleotide variant having at least 70% polynucleotide sequence identity to the polynucleotide encoding the polypeptide comprising an amino acid sequence selected from the group consisting of SEQ ID NO: 1-6 and fragments thereof.

Additionally, the invention provides an isolated and purified polynucleotide which hybridizes under stringent conditions to the polynucleotide encoding the polypeptide comprising an amino acid sequence selected from the group consisting of SEQ ID NO:1-6 and fragments thereof, as well as an isolated and purified polynucleotide having a sequence which is complementary to the polynucleotide encoding the polypeptide comprising the amino acid sequence selected from the group consisting of SEQ ID NO:1-6 and fragments thereof.

The invention also provides an isolated and purified polynucleotide comprising a polynucleotide sequence selected from the group consisting of SEQ ID NO:7-12 and fragments thereof. The invention further provides an isolated and purified polynucleotide variant having at least 90% polynucleotide sequence identity to the polynucleotide sequence comprising a polynucleotide sequence selected from the group consisting of SEQ ID NO:7-12 and fragments thereof, as well as an isolated and purified polynucleotide having a sequence which is complementary to the polynucleotide comprising a polynucleotide sequence selected from the group consisting of SEQ ID NO:7-12 and fragments thereof.

The invention further provides an expression vector containing at least a fragment of the polynucleotide encoding the polypeptide comprising an amino acid sequence selected from the group consisting of SEQ ID NO: 1-6 and fragments thereof. In another aspect, the expression vector is contained within a host cell.

The invention also provides a method for producing a polypeptide comprising the amino acid sequence selected from the group consisting of SEQ ID NO:1-6 and fragments thereof, the method comprising the steps of: (a) culturing the host cell containing an expression vector containing at least a fragment of a polynucleotide encoding the polypeptide under conditions suitable for the expression of the polypeptide; and (b) recovering the polypeptide from the host cell culture.

The invention also provides a pharmaceutical composition comprising a substantially purified polypeptide having the amino acid sequence selected from the group consisting of SEQ ID NO:1-6 and fragments thereof, in conjunction with a suitable pharmaceutical carrier.

The invention further includes a purified antibody which binds to a polypeptide comprising the amino acid sequence selected from the group consisting of SEQ ID NO:1-6 and fragments thereof, as well as a purified agonist and a purified antagonist to the polypeptide.

The invention also provides a method for treating or preventing a disorder associated with decreased expression or activity of HEPI, the method comprising administering to a subject in need of such treatment an effective amount of a pharmaceutical composition comprising a substantially purified polypeptide having an amino acid sequence selected from the group consisting of SEQ ID NO:1-6 and fragments thereof.

The invention also provides a method for treating or preventing a disorder associated with increased expression or activity of HEPI, the method comprising administering to a subject in need of such treatment an effective amount of an antagonist of the polypeptide having an amino acid sequence selected from the group consisting of SEQ ID NO: 1-6 and fragments thereof.

The invention also provides a method for detecting a polynucleotide encoding the polypeptide comprising the amino acid sequence selected from the group consisting of SEQ ID NO:1-6 and fragments thereof in a biological sample containing nucleic acids, the method comprising the steps of: (a) hybridizing the complement of the polynucleotide sequence encoding the polypeptide comprising the amino acid sequence selected from the group consisting of SEQ ID NO: 1-6 and fragments thereof to at least one of the nucleic acids of the biological sample, thereby forming a hybridization complex; and (b) detecting the hybridization complex, wherein the presence of the hybridization complex correlates with the presence of a polynucleotide encoding the polypeptide in the biological sample. In one aspect, the method further comprises amplifying the polynucleotide prior to hybridization.

## BRIEF DESCRIPTION OF THE FIGURES AND TABLES

Figure 1 shows the amino acid sequence alignments among HEPI-1 (2024646; SEQ ID NO:1), HEPI-2 (3431776; SEQ ID NO:2), HEPI-3 (1798487; SEQ ID NO:3), and human skin-specific protein (xp5) (GI 2589188; SEQ ID NO: 13), produced using the MEGALIGN program of LASERGENE software (DNASTAR Inc, Madison WI).

Figure 2 shows the amino acid sequence alignment between HEPI-4 (1448744; SEQ ID NO:4) and sheep keratin high-sulfur matrix protein IIIA3 (GI 71384; SEQ ID NO:14), produced using the multisequence alignment program of LASERGENE software (DNASTAR, Madison WI).

Figures 3A and 3B show the amino acid sequence alignment between HEPI-5 (2737275; SEQ ID NO:5) and mouse Ray protein (GI 1944389; SEQ ID NO:) 15), produced using the multisequence alignment program of LASERGENE software (DNASTAR, Madison WI).

Figures 4A, 4B, and 4C show the amino acid sequence alignment between HEPI-6 (3325323; SEQ ID NO:6) and human S protein (GI 414810; SEQ ID NO:16), produced using the multisequence alignment program of LASERGENE software (DNASTAR, Madison WI).

Table 1 shows the sequence identification numbers (SEQ ID NO:) of the amino acid and nucleic acid sequences, the Clone ID of the Incyte Clone in which nucleic acids encoding each HEPI were first identified, the cDNA library of the identifying clone, and the Incyte clones (and libraries) which are useful as fragments in hybridization technologies, and which are part of the consensus nucleotide sequence of each HEPI.

Table 2 shows various properties of the polypeptides of the invention: SEQ ID NO; the number of amino acid residues; potential phosphorylation sites; potential glycosylation sites; potential protein motifs or signature sequences; the identity of the protein; and analytical methods used to identify the protein through sequence homologies, protein motifs, and protein signatures.

Table 3 shows selected fragments of each nucleic acid sequence, the tissue expression of each nucleic acid sequence by northern analysis, diseases or conditions associated with this tissue expression, and the vector into which each cDNA was cloned.

Table 4 describes the tissues used to construct the cDNA libraries from which Incyte cDNA clones encoding HEPI were isolated.

Table 5 shows the programs, their descriptions, references, and threshold parameters used to analyze HEPI.

## DESCRIPTION OF THE INVENTION

Before the present proteins, nucleotide sequences, and methods are described, it is understood that this invention is not limited to the particular machines, materials and methods described, as these may vary. It is also to be understood that the terminology used herein is for the purpose of describing particular embodiments only, and is not intended to limit the scope of the present invention which will be limited only by the appended claims.
It must be noted that as used herein and in the appended claims, the singular forms "a," "an," and "the" include plural reference unless the context clearly dictates otherwise. Thus, for example, a reference to "a host cell" includes a plurality of such host cells, and a reference to "an antibody" is a reference to one or more antibodies and equivalents thereof known to those skilled in the art, and so forth.
Unless defined otherwise, all technical and scientific terms used herein have the same meanings as commonly understood by one of ordinary skill in the art to which this invention belongs. Although any machines, materials, and methods similar or equivalent to those described herein can be used to practice or test the present invention, the preferred machines, materials and methods are now described. All publications mentioned herein are cited for the purpose of describing and disclosing the cell lines, protocols, reagents and vectors which are reported in the publications and which might be used in connection with the invention. Nothing herein is to be construed as an admission that the invention is not entitled to antedate such disclosure by virtue of prior invention.

## DEFINITIONS

"HEPI" refers to the amino acid sequences of substantially purified HEPI obtained from any species, particularly a mammalian species, including bovine, ovine, porcine, murine, equine, and preferably the human species, from any source, whether natural, synthetic, semi-synthetic, or recombinant.
The term "agonist" refers to a molecule which, when bound to HEPI, increases or prolongs the duration of the effect of HEPI. Agonists may include proteins, nucleic acids, carbohydrates, or any other molecules which bind to and modulate the effect of HEPI.
An "allelic variant" is an alternative form of the gene encoding HEPI. Allelic variants may result from at least one mutation in the nucleic acid sequence and may result in altered mRNAs or in polypeptides whose structure or function may or may not be altered. Any given natural or recombinant gene may have none, one, or many allelic forms. Common mutational changes which give rise to allelic variants are generally ascribed to natural deletions, additions, or substitutions of nucleotides. Each of these types of changes may occur alone, or in combination with the others, one or more times in a given sequence.
"Altered" nucleic acid sequences encoding HEPI include those sequences with deletions, insertions, or substitutions of different nucleotides, resulting in a polynucleotide the same as HEPI or a polypeptide with at least one functional characteristic of HEPI. Included within this definition are polymorphisms which may or may not be readily detectable using a particular oligonucleotide probe of the polynucleotide encoding HEPI, and improper or unexpected hybridization to allelic variants, with a locus other than the normal chromosomal locus for the polynucleotide sequence encoding HEPI. The encoded protein may also be "altered," and may contain deletions, insertions, or substitutions of amino acid residues which produce a silent change and result in a functionally equivalent HEPI. Deliberate amino acid substitutions may be made on the basis of similarity in polarity, charge, solubility, hydrophobicity, hydrophilicity, and/or the amphipathic nature of the residues, as long as the biological or immunological activity of HEPI is retained. For example, negatively charged amino acids may include aspartic acid and glutamic acid, positively charged amino acids may include lysine and arginine, and amino acids with uncharged polar head groups having similar hydrophilicity values may include leucine, isoleucine, and valine; glycine and alanine; asparagine and glutamine; serine and threonine; and phenylalanine and tyrosine.
The terms "amino acid" or "amino acid sequence" refer to an oligopeptide, peptide, polypeptide, or protein sequence, or a fragment of any of these, and to naturally occurring or synthetic molecules. In this context, "fragments," "immunogenic fragments," or "antigenic fragments" refer to fragments of HEPI which are preferably at least 5 to about 15 amino acids in length, most preferably at least 14 amino acids, and which retain some biological activity or immunological activity of HEPI. Where "amino acid sequence" is recited to refer to an amino acid sequence of a naturally occurring protein molecule, "amino acid sequence" and like terms are not meant to limit the amino acid sequence to the complete native amino acid sequence associated with the recited protein molecule.
"Amplification" relates to the production of additional copies of a nucleic acid sequence. Amplification is generally carried out using polymerase chain reaction (PCR) technologies well known in the art.
The term "antagonist" refers to a molecule which, when bound to HEPI, decreases the amount or the duration of the effect of the biological or immunological activity of HEPI. Antagonists may include proteins, nucleic acids, carbohydrates, antibodies, or any other molecules which decrease the effect of HEPI.
The term "antibody" refers to intact molecules as well as to fragments thereof, such as Fab, $F(ab')_2$, and Fv

fragments, which are capable of binding the epitopic determinant. Antibodies that bind HEPI polypeptides can be prepared using intact polypeptides or using fragments containing small peptides of interest as the immunizing antigen. The polypeptide or oligopeptide used to immunize an animal (e.g., a mouse, a rat, or a rabbit) can be derived from the translation of RNA, or synthesized chemically, and can be conjugated to a carrier protein if desired. Commonly used carriers that are chemically coupled to peptides include bovine serum albumin, thyroglobulin, and keyhole limpet hemocyanin (KLH). The coupled peptide is then used to immunize the animal.

The term "antigenic determinant" refers to that fragment of a molecule (i.e., an epitope) that makes contact with a particular antibody. When a protein or a fragment of a protein is used to immunize a host animal, numerous regions of the protein may induce the production of antibodies which bind specifically to antigenic determinants (given regions or three-dimensional structures on the protein). An antigenic determinant may compete with the intact antigen (i.e., the immunogen used to elicit the immune response) for binding to an antibody.

The term "antisense" refers to any composition containing a nucleic acid sequence which is complementary to the "sense" strand of a specific nucleic acid sequence. Antisense molecules may be produced by any method including synthesis or transcription. Once introduced into a cell, the complementary nucleotides combine with natural sequences produced by the cell to form duplexes and to block either transcription or translation. The designation "negative" can refer to the antisense strand, and the designation "positive" can refer to the sense strand.

The term "biologically active," refers to a protein having structural, regulatory, or biochemical functions of a naturally occurring molecule. Likewise, "immunologically active" refers to the capability of the natural, recombinant, or synthetic HEPI, or of any oligopeptide thereof, to induce a specific immune response in appropriate animals or cells and to bind with specific antibodies.

The terms "complementary" or "complementarity" refer to the natural binding of polynucleotides by base pairing. For example, the sequence "5' A-G-T 3"' bonds to the complementary sequence "3' T-C-A 5'." Complementarity between two single-stranded molecules may be "partial," such that only some of the nucleic acids bind, or it may be "complete," such that total complementarity exists between the single stranded molecules. The degree of complementarity between nucleic acid strands has significant effects on the efficiency and strength of the hybridization between the nucleic acid strands. This is of particular importance in amplification reactions, which depend upon binding between nucleic acids strands, and in the design and use of peptide nucleic acid (PNA) molecules.

A "composition comprising a given polynucleotide sequence" or a "composition comprising a given amino acid sequence" refer broadly to any composition containing the given polynucleotide or amino acid sequence. The composition may comprise a dry formulation or an aqueous solution. Compositions comprising polynucleotide sequences encoding HEPI or fragments of HEPI may be employed as hybridization probes. The probes may be stored in freeze-dried form and may be associated with a stabilizing agent such as a carbohydrate. In hybridizations, the probe may be deployed in an aqueous solution containing salts (e.g., NaCl), detergents (e.g., sodium dodecyl sulfate; SDS), and other components (e.g., Denhardt's solution, dry milk, salmon sperm DNA, etc.).

"Consensus sequence"refers to a nucleic acid sequence which has been resequenced to resolve uncalled bases, extended using XL-PCR kit (Perkin-Elmer, Norwalk CT) in the 5' and/or the 3' direction, and resequenced, or which has been assembled from the overlapping sequences of more than one Incyte Clone using a computer program for fragment assembly, such as the GELVIEW Fragment Assembly system (GCG, Madison WI). Some sequences have been both extended and assembled to produce the consensus sequence.

The term "correlates with expression of a polynucleotide" indicates that the detection of the presence of nucleic acids, the same or related to a nucleic acid sequence encoding HEPI, by northern analysis is indicative of the presence of nucleic acids encoding HEPI in a sample, and thereby correlates with expression of the transcript from the polynucleotide encoding HEPI.

A "deletion" refers to a change in the amino acid or nucleotide sequence that results in the absence of one or more amino acid residues or nucleotides.

The term "derivative" refers to the chemical modification of a polypeptide sequence, or a polynucleotide sequence. Chemical modifications of a polynucleotide sequence can include, for example, replacement of hydrogen by an alkyl, acyl, or amino group. A derivative polynucleotide encodes a polypeptide which retains at least one biological or immunological function of the natural molecule. A derivative polypeptide is one modified by glycosylation, pegylation, or any similar process that retains at least one biological or immunological function of the polypeptide from which it was derived.

The term "similarity" refers to a degree of complementarity. There may be partial similarity or complete similarity. The word "identity" may substitute for the word "similarity." A partially complementary sequence that at least partially inhibits an identical sequence from hybridizing to a target nucleic acid is referred to as "substantially similar." The inhibition of hybridization of the completely complementary sequence to the target sequence may be examined using a hybridization assay (Southern or northern blot, solution hybridization, and the like) under conditions of reduced stringency. A substantially similar sequence or hybridization probe will compete for and inhibit the binding of a completely similar (identical) sequence to the target sequence under conditions of reduced stringency. This is not to say that conditions of reduced stringency are such that non-specific binding is permitted, as reduced stringency conditions require that the binding of two sequences to one another be a specific (i.e., a selective) interaction. The absence of non-specific binding may be tested by the use of a second target sequence which lacks even a partial degree of complementarity (e.g., less than about 30% similarity or identity). In the absence of non-specific binding, the substantially similar sequence or probe will not hybridize to the second non-complementary target sequence.

The phrases "percent identity" and "% identity" refer to the percentage of sequence similarity found in a comparison of two or more amino acid or nucleic acid sequences. Percent identity can be determined electronically, e.g., by using the MEGALIGN program (DNASTAR, Madison WI) which creates alignments between two or more sequences according to methods selected by the user, e.g., the clustal method. (See, e.g., Higgins, D.G. and P.M. Sharp (1988) Gene 73:237-244.) The clustal algorithm groups sequences into clusters by examining the distances between all pairs. The clusters are aligned pairwise and then in groups. The percentage similarity between two amino acid sequences, e.g., sequence A and sequence B, is calculated by dividing the length of sequence A, minus the number of gap residues in sequence A, minus the number of gap residues in sequence B, into the sum of the residue matches between sequence A and sequence B, times one hundred. Gaps of low or of no similarity between the two amino acid sequences are not included in determining percentage similarity. Percent identity between nucleic acid sequences can also be counted or calculated by other methods known in the art, e.g., the Jotun Hein method. (See, e.g., Hein, J. (1990) Methods Enzymol. 183:626-645.) Identity between sequences can also be determined by other methods known in the art, e.g., by varying hybridization conditions.

"Human artificial chromosomes" (HACs) are linear microchromosomes which may contain DNA sequences of about 6 kb to 10 Mb in size, and which contain all of the elements required for stable mitotic chromosome segregation and maintenance.

The term "humanized antibody" refers to antibody molecules in which the amino acid sequence in the non-antigen binding regions has been altered so that the antibody more closely resembles a human antibody, and still retains its original binding ability.

"Hybridization" refers to any process by which a strand of nucleic acid binds with a complementary strand through base pairing.

The term "hybridization complex" refers to a complex formed between two nucleic acid sequences by virtue of the formation of hydrogen bonds between complementary bases. A hybridization complex may be formed in solution (e.g., $C_ot$ or $R_ot$ analysis) or formed between one nucleic acid sequence present in solution and another nucleic acid sequence immobilized on a solid support (e.g., paper, membranes, filters, chips, pins or glass slides, or any other appropriate substrate to which cells or their nucleic acids have been fixed).

The words "insertion" or "addition" refer to changes in an amino acid or nucleotide sequence resulting in the addition of one or more amino acid residues or nucleotides, respectively, to the sequence found in the naturally occurring molecule. "Immune response" can refer to conditions associated with inflammation, trauma, immune disorders, or infectious or genetic disease, etc. These conditions can be characterized by expression of various factors, e.g., cytokines, chemokines, and other signaling molecules, which may affect cellular and systemic defense systems.

The term "microarray" refers to an arrangement of distinct polynucleotides on a substrate.

The terms "element" or "array element" in a microarray context, refer to hybridizable polynucleotides arranged on the surface of a substrate.

The term "modulate" refers to a change in the activity of HEPI. For example, modulation may cause an increase or a decrease in protein activity, binding characteristics, or any other biological, functional, or immunological properties of HEPI.

The phrases "nucleic acid" or "nucleic acid sequence," as used herein, refer to a nucleotide, oligonucleotide, polynucleotide, or any fragment thereof. These phrases also refer to DNA or RNA of genomic or synthetic origin which may be single-stranded or double-stranded and may represent the sense or the antisense strand, to peptide nucleic acid (PNA), or to any DNA-like or RNA-like material. In this context, "fragments" refers to those nucleic acid sequences which, comprise a region of unique polynucleotide sequence that specifically identifies SEQ ID NO:7-12, for example, as distinct from any other sequence in the same genome. For example, a fragment of SEQ ID NO:7-12 is useful in hybridization and amplification technologies and in analogous methods that distinguish SEQ ID NO:7-12 from related polynucleotide sequences. A fragment of SEQ ID NO:7-12 is at least about 15-20 nucleotides in length. The precise length of the fragment of SEQ ID NO:7-12 and the region of SEQ ID NO:7-12 to which the fragment corresponds are routinely determinable by one of ordinary skill in the art based on the intended purpose for the fragment. In some cases, a fragment, when translated, would produce polypeptides retaining some functional characteristic, e.g., antigenicity, or structural domain characteristic, e.g., ATP-binding site, of the full-length polypeptide.

The terms "operably associated" or "operably linked" refer to functionally related nucleic acid sequences. A promoter is operably associated or operably linked with a coding sequence if the promoter controls the translation of the encoded polypeptide. While operably associated or operably linked nucleic acid sequences can be contiguous and in the same reading frame, certain genetic elements, e.g., repressor genes, are not contiguously linked to the sequence encoding the polypeptide but still bind to operator sequences that control expression of the polypeptide.

The term "oligonucleotide" refers to a nucleic acid sequence of at least about 6 nucleotides to 60 nucleotides, preferably about 15 to 30 nucleotides, and most preferably about 20 to 25 nucleotides, which can be used in PCR amplification or in a hybridization assay or microarray. "Oligonucleotide" is substantially equivalent to the terms "amplimer," "primer," "oligomer," and "probe," as these terms are commonly defined in the art.

"Peptide nucleic acid" (PNA) refers to an antisense molecule or anti-gene agent which comprises an oligonucleotide of at least about 5 nucleotides in length linked to a peptide backbone of amino acid residues ending in lysine. The terminal lysine confers solubility to the composition. PNAs preferentially bind complementary single stranded DNA or RNA and stop transcript elongation, and may be pegylated to extend their lifespan in the cell.

The term "sample" is used in its broadest sense. A sample suspected of containing nucleic acids encoding HEPI, or fragments thereof, or HEPI itself, may comprise a bodily fluid; an extract from a cell, chromosome, organelle, or membrane isolated from a cell; a cell; genomic DNA, RNA, or cDNA, in solution or bound to a substrate; a tissue; a tissue print; etc.

The terms "specific binding" or "specifically binding" refer to that interaction between a protein or peptide and an agonist, an antibody, or an antagonist. The interaction is dependent upon the presence of a particular structure of the protein, e.g., the antigenic determinant or epitope. recognized by the binding molecule. For example, if an antibody is specific for epitope "A," the presence of a polypeptide containing the epitope A, or the presence of free unlabeled A, in a reaction containing free labeled A and the antibody will reduce the amount of labeled A that binds to the antibody.

The term "stringent conditions" refers to conditions which permit hybridization between polynucleotides and the claimed polynucleotides. Stringent conditions can be defined by salt concentration, the concentration of organic solvent, e.g., formamide, temperature, and other conditions well known in the art. In particular, stringency can be increased by reducing the concentration of salt, increasing the concentration of formamide, or raising the hybridization temperature.

The term "substantially purified" refers to nucleic acid or amino acid sequences that are removed from their natural environment and are isolated or separated, and are at least about 60% free, preferably about 75% free, and most preferably about 90% free from other components with which they are naturally associated.

A "substitution" refers to the replacement of one or more amino acids or nucleotides by different amino acids or nucleotides, respectively.

"Substrate" refers to any suitable rigid or semi-rigid support including membranes, filters, chips, slides, wafers, fibers, magnetic or nonmagnetic beads, gels, tubing, plates, polymers, microparticles and capillaries. The substrate can have a variety of surface forms, such as wells, trenches, pins, channels and pores, to which polynucleotides or polypeptides are bound.

"Transformation" describes a process by which exogenous DNA enters and changes a recipient cell. Transformation may occur under natural or artificial conditions according to various methods well known in the art, and may rely on any known method for the insertion of foreign nucleic acid sequences into a prokaryotic or eukaryotic host cell. The method for transformation is selected based on the type of host cell being transformed and may include, but is not limited to, viral infection, electroporation, heat shock, lipofection, and particle bombardment. The term "transformed" cells includes stably transformed cells in which the inserted DNA is capable of replication either as an autonomously replicating plasmid or as part of the host chromosome, as well as transiently transformed cells which express the inserted DNA or RNA for limited periods of time.

A "variant" of HEPI polypeptides refers to an amino acid sequence that is altered by one or more amino acid residues. The variant may have "conservative" changes, wherein a substituted amino acid has similar structural or chemical properties (e.g., replacement of leucine with isoleucine). More rarely, a variant may have "nonconservative" changes (e.g., replacement of glycine with tryptophan). Analogous minor variations may also include amino acid deletions or insertions, or both. Guidance in determining which amino acid residues may be substituted, inserted, or deleted without abolishing biological or immunological activity may be found using computer programs well known in the art, for example, LASERGENE software (DNASTAR).

The term "variant," when used in the context of a polynucleotide sequence, may encompass a polynucleotide sequence related to KEPI. This definition may also include, for example, "allelic" (as defined above), "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs) in which the polynucleotide sequence varies by one base. The presence of SNPs may be indicative of, for example, a certain population, a disease state, or a propensity for a disease state.

## THE INVENTION

The invention is based on the discovery of new human epidermal proteins (HEPI), the polynucleotides encoding HEPI, and the use of these compositions for the diagnosis, treatment, or prevention of epithelial, cell proliferative, and autoimmune/inflammatory disorders. Table 1 summarizes the sequence identification numbers, identifying clone numbers, and libraries of HEPI.

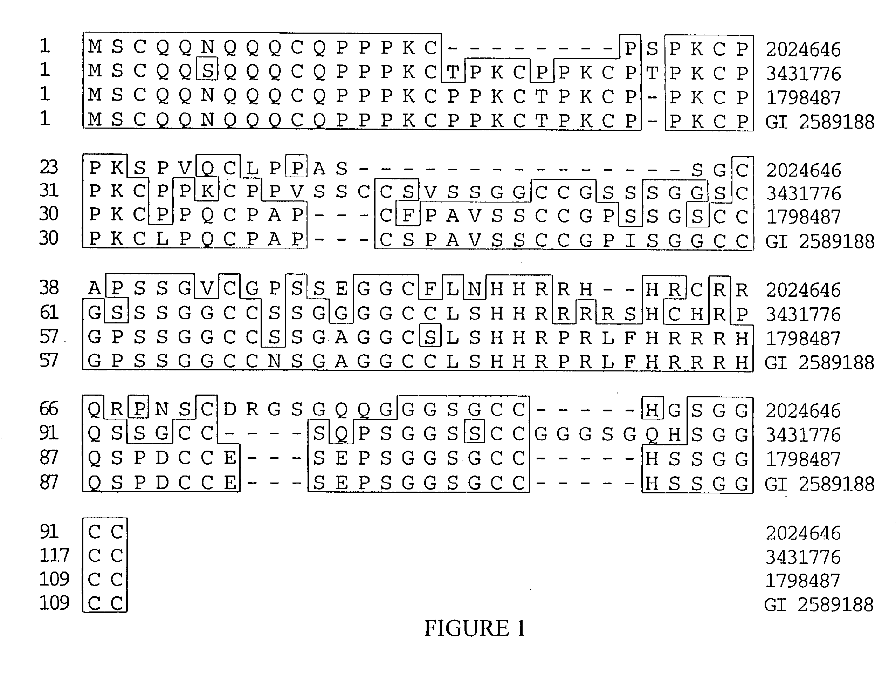

As shown in Table 2, each HEPI has been characterized with regard to its chemical and structural similarity with epithelial proteins. As shown in Figure 1, HEPI-1 and human skin specific protein (xp5) (GI 2589188; SEQ ID NO: 13) share 53% identity, HEPI-2 and human skin specific protein (xp5) (GI 2589188; SEQ ID NO:13) share 61 % identity, and HEPI-3 and human skin specific protein (xp5) (GI 2589188; SEQ ID NO:13) share 95% identity. HEPI-1,HEPI-2, HEPI-3, and human skin-specific protein (GI 2589188; SEQ ID NO:13) have similar molecular weights of 9532, 11,545, 11,225, and 11.220, respectively. PRINTS analysis indicates that HEPI-1, HEPI-2, and HEPI-3 contain sequences similar to those of small proline-rich proteins (PR00021).

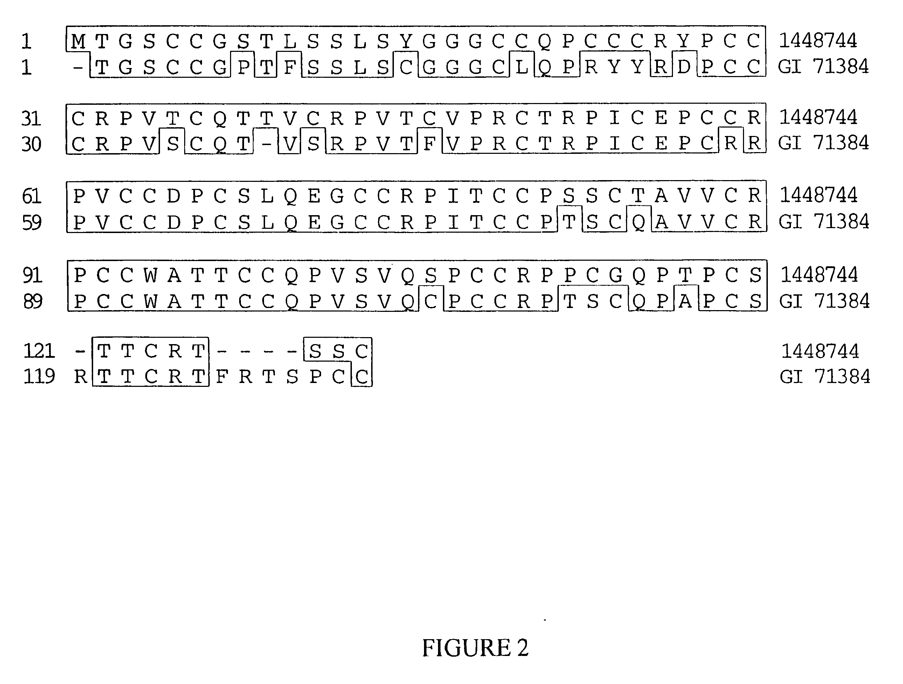

HEPI-4 contains 28 mol % cysteine. As shown in Figure 2, HEPI-4 has chemical and structural similarity with sheep keratin high-sulfur matrix protein IIIA3 (GI 71384; SEQ ID NO:14). In particular, HEPI-4 and sheep keratin high-sulfur matrix protein IIIA3 share 77% identity.

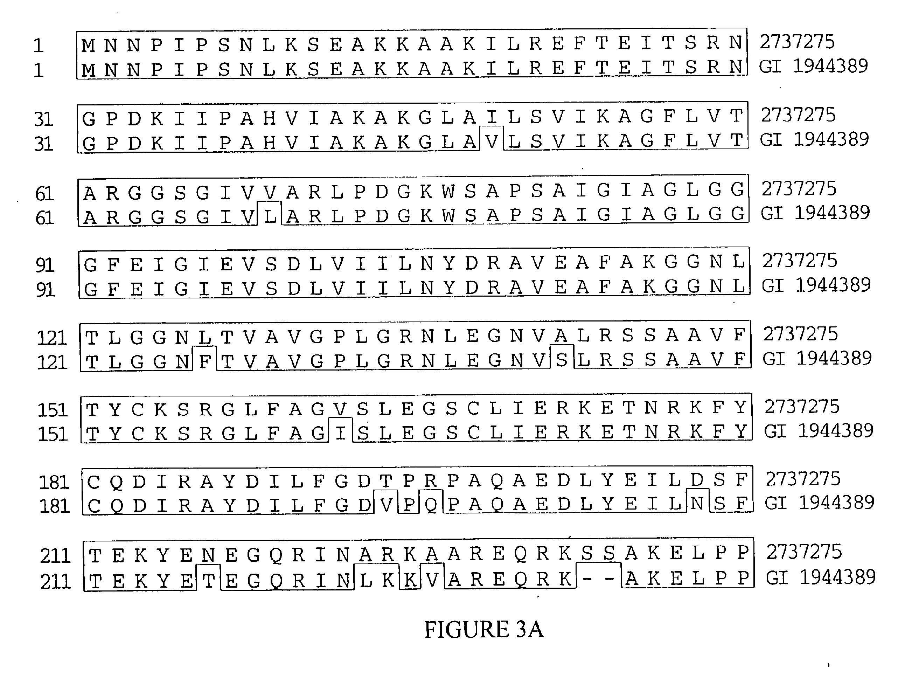

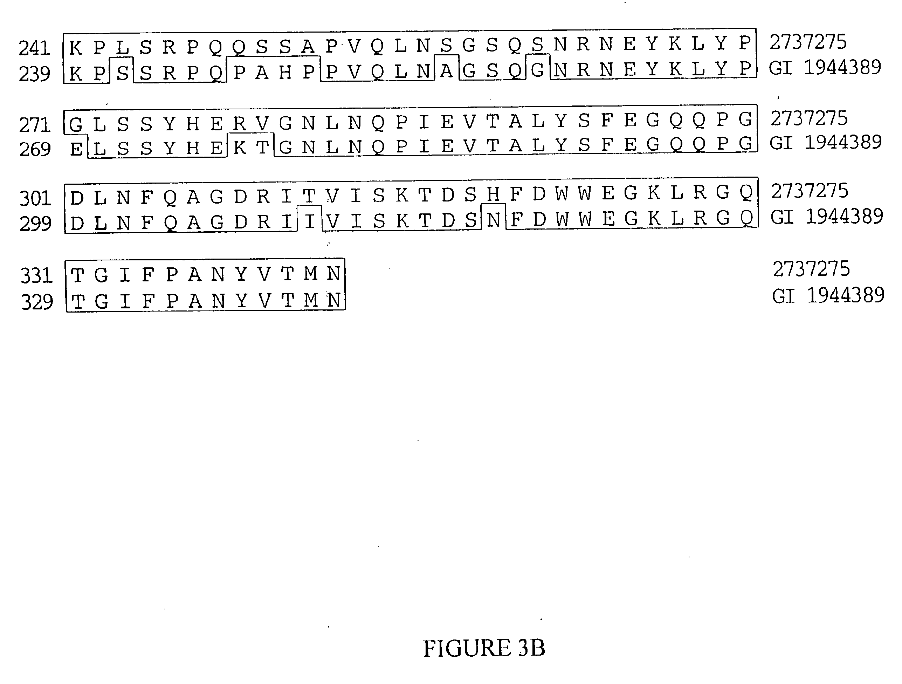

As shown in Figures 3A and 3B, HEPI-5 has chemical and structural similarity with mouse Ray, a protein expressed in skin (GI 1944389; SEQ ID NO:15). In particular, HEPI-2 and mouse Ray share 92% identity.

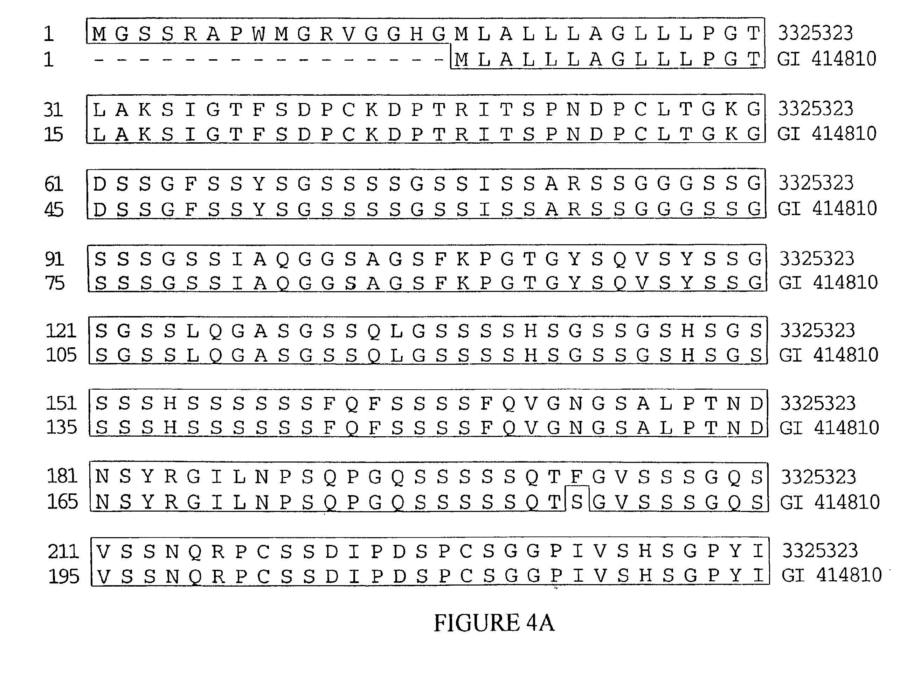

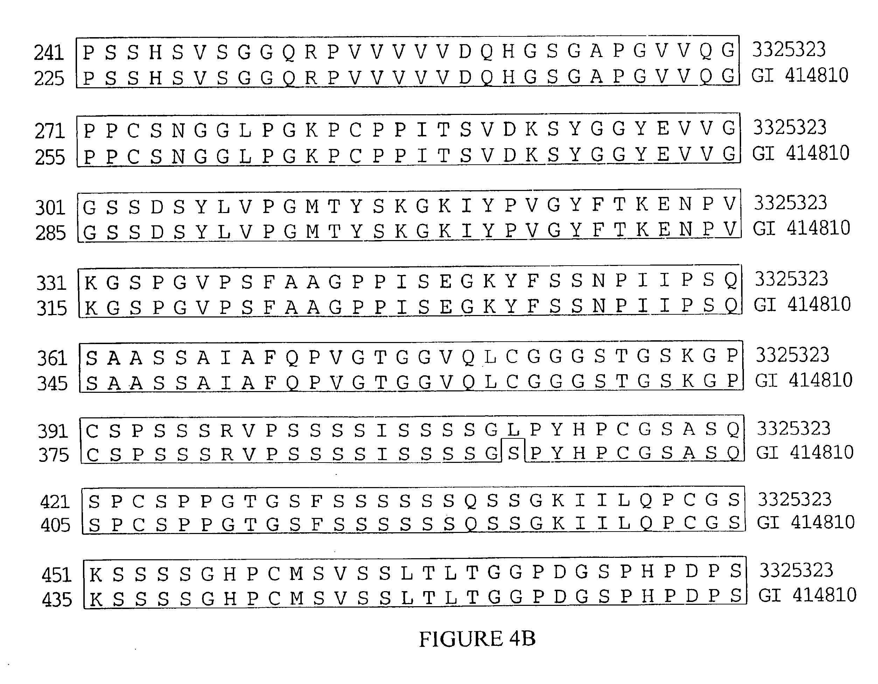

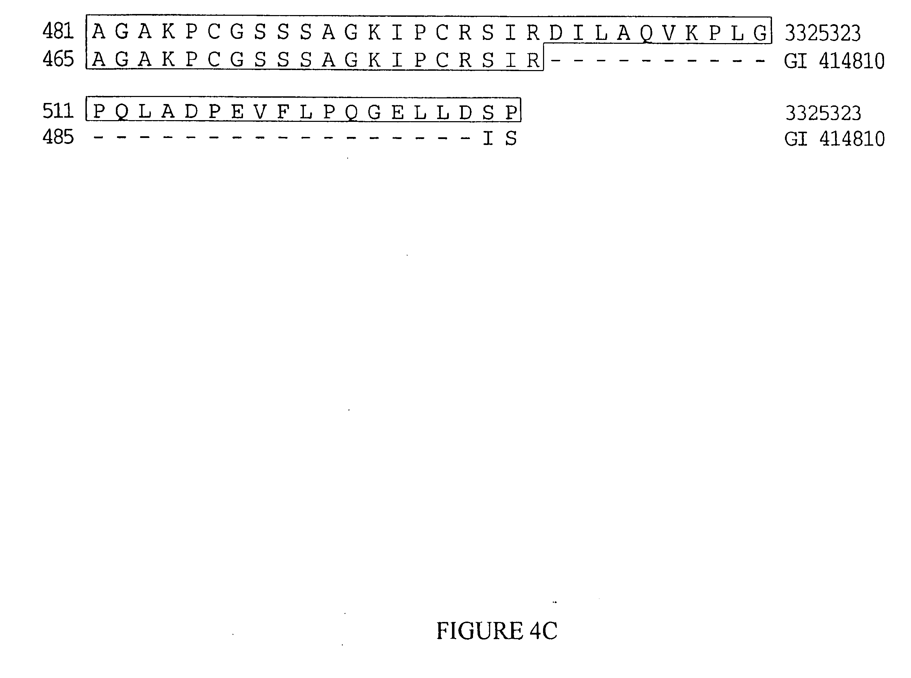

HEPI-6 contains 27 mol % serine, 16 mol % glycine, and 10 mol % proline. As shown in Figures 4A, 4B, and 4C, HEPI-6 has chemical and structural similarity with human S protein (GI 414810; SEQ ID NO:16). In particular, residue M17 through residue 1502 of HEPI-6 and human S protein share 99% identity. HEPI-6 contains 16 additional N-terminal and 27 additional C-terminal residues not present in human S protein.

Table 3 shows the tissue-specificity and diseases, disorders, or conditions associated with nucleotide sequences encoding HEPI. The first column of Table 3 lists the nucleotide SEQ ID NOs. Column 2 lists fragments of the nucleotide sequences of column 1. These fragments are useful, for example, in hybridization or amplification technologies to identify SEQ ID NO:7-12 and to distinguish between SEQ ID NO:7-12 and related polynucleotide sequences. The polypeptides encoded by these fragments are useful, for example, as immunogenic peptides. Column 3 lists tissue categories which express HEPI as a fraction of total tissues expressing HEPI. Column 4 lists diseases, disorders, or conditions associated with those tissues expressing HEPI as a fraction of total tissues expressing HEPI. Column 5 lists the vectors used to subclone each cDNA library. Of particular note is the expression of HEPI-1, HEPI-2, and HEPI-3 in proliferating skin tissues, the expression of HEPI-5 in epidermal keratinocytes, dermal fibroblasts, and bronchial epithelium, and the expression of HEPI-6 in breast skin and in keratinocytes. Northern analysis shows the expression of HEPI-4 in two libraries, both of which are associated with cell proliferation, one of which is associated with inflammation and immune response, and one of which is a keratinocyte cell line.

The invention also encompasses HEPI variants. A preferred HEPI variant is one which has at least about 80%, more preferably at least about 90%, and most preferably at least about 95% amino acid sequence identity to the HEPI amino acid sequence, and which contains at least one functional or structural characteristic of HEPI. The invention also encompasses polynucleotides which encode HEPI. In a particular embodiment, the invention encompasses a polynucleotide sequence comprising a sequence selected from the group consisting of SEQ ID NO:7-12 which encodes HEPI.

The invention also encompasses a variant of a polynucleotide sequence encoding HEPI. In particular, such a variant polynucleotide sequence will have at least about 70%, more preferably at least about 85%, and most preferably at least about 95% polynucleotide sequence identity to the polynucleotide sequence encoding HEPI. A particular aspect of the invention encompasses a variant of a polynucleotide sequence comprising a sequence selected from the group consisting of SEQ ID NO:7-12 which has at least about 70%, more preferably at least about 85%, and most preferably at least about 95% polynucleotide sequence identity to a nucleic acid sequence selected from the group consisting of SEQ ID NO:7-12. Any one of the polynucleotide variants described above can encode an amino acid sequence which contains at least one functional or structural characteristic of HEPI.

It will be appreciated by those skilled in the art that as a result of the degeneracy of the genetic code, a multitude of polynucleotide sequences encoding HEPI, some bearing minimal similarity to the polynucleotide sequences of any known and naturally occurring gene, may be produced. Thus, the invention contemplates each and every possible variation of polynucleotide sequence that could be made by selecting combinations based on possible codon choices. These combinations are made in accordance with the standard triplet genetic code as applied to the polynucleotide sequence of naturally occurring HEPI, and all such variations are to be considered as being specifically disclosed.

Although nucleotide sequences which encode HEPI are preferably capable of hybridizing to the nucleotide sequence of the naturally occurring HEPI under appropriately selected conditions of stringency, it may be advantageous to produce nucleotide sequences encoding HEPI or its derivatives possessing a substantially different codon usage, e.g., inclusion of non-naturally occurring codons. Codons may be selected to increase the rate at which expression of the peptide occurs in a particular prokaryotic or eukaryotic host in accordance with the frequency with which particular codons are utilized by the host. Other reasons for substantially altering the nucleotide sequence encoding HEPI and its derivatives without altering the encoded amino acid sequences include the production of RNA transcripts having more desirable properties, such as a greater half-life, than transcripts produced from the naturally occurring sequence.

The invention also encompasses production of DNA sequences which encode HEPI and HEPI derivatives, or fragments thereof, entirely by synthetic chemistry. After production, the synthetic sequence may be inserted into any of the many available expression vectors and cell systems using reagents well known in the art. Moreover, synthetic chemistry may be used to introduce mutations into a sequence encoding HEPI or any fragment thereof.

Also encompassed by the invention are polynucleotide sequences that are capable of hybridizing to the claimed polynucleotide sequences, and, in particular, to those shown in SEQ ID NO:7-12 and fragments thereof under various conditions of stringency. (See, e.g., Wahl, G.M. and S.L. Berger (1987) Methods Enzymol. 152:399-407; Kimmel, A.R. (1987) Methods Enzymol. 152:507-511.) For example, stringent salt concentration will ordinarily be less than about 750 mM NaCl and 75 mM trisodium citrate, preferably less than about 500 mM NaCl and 50 mM trisodium citrate, and most preferably less than about 250 mM NaCl and 25 mM trisodium citrate. Low stringency hybridization can be obtained in the absence of organic solvent, e.g., formamide, while high stringency hybridization can be obtained in the presence of at least about 35% formamide, and most preferably at least about 50% formamide. Stringent temperature conditions will ordinarily include temperatures of at least about 30°C, more preferably of at least about 37°C, and most preferably of at least about 42°C. Varying additional parameters, such as hybridization time, the concentration of detergent, e.g., sodium dodecyl sulfate (SDS), and the inclusion or exclusion of carrier DNA, are well known to those skilled in the art. Various levels of stringency are accomplished by combining these various conditions as needed. In a preferred embodiment, hybridization will occur at 30°C in 750 mM NaCl, 75 mM trisodium citrate, and 1% SDS. In a more preferred embodiment, hybridization will occur at 37°C in 500 mM NaCl, 50 mM trisodium citrate, 1% SDS, 35% formamide, and 100 μg/ml denatured salmon sperm DNA (ssDNA). In a most preferred embodiment, hybridization will occur at 42°C in 250 mM NaCl, 25 mM trisodium citrate, 1% SDS. 50 % formamide, and 200 μg/ml ssDNA. Useful variations on these conditions will be readily apparent to those skilled in the art.

The washing steps which follow hybridization can also vary in stringency. Wash stringency conditions can be defined by salt concentration and by temperature. As above, wash stringency can be increased by decreasing salt concentration or by increasing temperature. For example, stringent salt concentration for the wash steps will preferably be less than about 30 mM NaCl and 3 mM trisodium citrate, and most preferably less than about 15 mM NaCl and 1.5 mM trisodium citrate. Stringent temperature conditions for the wash steps will ordinarily include temperature of at least about 25°C, more preferably of at least about 42°C, and most preferably of at least about 68°C. In a preferred embodiment, wash steps will occur at 25°C in 30 mM NaCl, 3 mM trisodium citrate, and 0.1% SDS. In a more preferred embodiment, wash steps will occur at 42°C in 15 mM NaCl, 1.5 mM trisodium citrate, and 0.1% SDS. In a most preferred embodiment, wash steps will occur at 68°C in 15 mM NaCl, 1.5 mM trisodium citrate, and 0.1% SDS. Additional variations on these conditions will be readily apparent to those skilled in the art.

Methods for DNA sequencing are well known in the art and may be used to practice any of the embodiments of the invention. The methods may employ such enzymes as the Klenow fragment of DNA polymerase I, SEQUENASE (US Biochemical, Cleveland OH), Taq polymerase (Perkin-Elmer), thermostable T7 polymerase (Amersham Pharmacia Biotech, Piscataway NJ), or combinations of polymerases and proofreading exonucleases such as those found in the ELONGASE amplification system (Life Technologies, Gaithersburg MD). Preferably, sequence preparation is automated with machines such as the Hamilton MICROLAB 2200 (Hamilton, Reno NV), Peltier Thermal Cycler 200 (PTC200; MJ Research, Watertown MA) and the ABI CATALYST 800 (Perkin-Elmer). Sequencing is then carried out using either ABI 373 or 377 DNA sequencing systems (Perkin-Elmer) or the MEGABACE 1000 DNA sequencing system (Molecular Dynamics, Sunnyvale CA). The resulting sequences are analyzed using a variety of algorithms which are well known in the art. (See, e.g., Ausubel, F.M. (1997) Short Protocols in Molecular Biology, John Wiley & Sons, New York NY, unit 7.7; Meyers, R.A. (1995) Molecular Biology and Biotechnology, Wiley VCH, New York NY, pp. 856-853.)

The nucleic acid sequences encoding HEPI may be extended utilizing a partial nucleotide sequence and employing various PCR-based methods known in the art to detect upstream sequences, such as promoters and regulatory elements. For example, one method which may be employed, restriction-site PCR, uses universal and nested primers to amplify unknown sequence from genomic DNA within a cloning vector. (See, e.g., Sarkar, G. (1993) PCR Methods Applic. 2:318-322.) Another method, inverse PCR, uses primers that extend in divergent directions to amplify unknown sequence from a circularized template. The template is derived from restriction fragments comprising a known genomic locus and surrounding sequences. (See, e.g., Triglia, T. et al. (1988) Nucleic Acids Res. 16:8186.) A third method, capture PCR, involves PCR amplification of DNA fragments adjacent to known sequences in human and yeast artificial chromosome DNA. (See, e.g., Lagerstrom, M. et al. (1991) PCR Methods Applic. 1:111-119.) In this method, multiple restriction enzyme digestions and ligations may be used to insert an engineered double-stranded sequence into a region of unknown sequence before performing PCR. Other methods which may be used to retrieve unknown sequences are known in the art. (See, e.g., Parker, J.D. et al. (1991) Nucleic Acids Res. 19:3055-306). Additionally, one may use PCR, nested primers, and PROMOTERFINDER libraries (Clontech, Palo Alto CA) to walk genomic DNA. This procedure avoids the need to screen libraries and is useful in finding intron/exon junctions. For all PCR-based methods, primers may be designed using commercially available software, such as OLIGO 4.06 Primer Analysis software (National Biosciences, Plymouth MN) or another appropriate program, to be about 22 to 30 nucleotides in length, to have a GC content of about 50% or more, and to anneal to the template at temperatures of about 68°C to 72°C.

When screening for full-length cDNAs, it is preferable to use libraries that have been size-selected to include larger cDNAs. In addition, random-primed libraries, which often include sequences containing the 5' regions of genes, are preferable for situations in which an oligo d(T) library does not yield a full-length cDNA. Genomic libraries may be useful for extension of sequence into 5' non-transcribed regulatory regions.

Capillary electrophoresis systems which are commercially available may be used to analyze the size or confirm the nucleotide sequence of sequencing or PCR products. In particular, capillary sequencing may employ flowable polymers for electrophoretic separation, four different nucleotide-specific, laser-stimulated fluorescent dyes, and a charge coupled device camera for detection of the emitted wavelengths. Output/light intensity may be converted to electrical signal using appropriate software (e.g., GENOTYPER and SEQUENCE NAVIGATOR, Perkin-Elmer), and the entire process from loading of samples to computer analysis and electronic data display may be computer controlled. Capillary electrophoresis is especially preferable for sequencing small DNA fragments which may be present in limited amounts in a particular sample.

In another embodiment of the invention, polynucleotide sequences or fragments thereof which encode HEPI may be cloned in recombinant DNA molecules that direct expression of HEPI, or fragments or functional equivalents thereof, in appropriate host cells. Due to the inherent degeneracy of the genetic code, other DNA sequences which encode substantially the same or a functionally equivalent amino acid sequence may be produced and used to express HEPI.

The nucleotide sequences of the present invention can be engineered using methods generally known in the art in order to alter HEPI-encoding sequences for a variety of purposes including, but not limited to, modification of the cloning, processing, and/or expression of the gene product. DNA shuffling by random fragmentation and PCR reassembly of gene fragments and synthetic oligonucleotides may be used to engineer the nucleotide sequences. For example, oligonucleotide-mediated site-directed mutagenesis may be used to introduce mutations that create new restriction sites, alter glycosylation patterns, change codon preference, produce splice variants, and so forth.

In another embodiment, sequences encoding HEPI may be synthesized, in whole or in part, using chemical methods well known in the art. (See, e.g., Caruthers, M.H. et al. (1980) Nucl. Acids Res. Symp. Ser. 215-223, and Horn, T. et al. (1980) Nucl. Acids Res. Symp. Ser. 225-232.) Alternatively, HEPI itself or a fragment thereof may be synthesized using chemical methods. For example, peptide synthesis can be performed using various solid-phase techniques. (See, e.g., Roberge, J.Y. et al. (1995) Science 269:202-204.) Automated synthesis may be achieved using the ABI 431A Peptide Synthesizer (Perkin-Elmer). Additionally, the amino acid sequence of HEPI, or any part thereof, may be altered during direct synthesis and/or combined with sequences from other proteins, or any part thereof, to produce a variant polypeptide.

The peptide may be substantially purified by preparative high performance liquid chromatography. (See, e.g, Chiez, R.M. and F.Z. Regnier (1990) Methods Enzymol. 182:392-421.) The composition of the synthetic peptides may be confirmed by amino acid analysis or by sequencing. (See, e.g., Creighton, T. (1984) Proteins, Structures and Molecular Properties, WH Freeman, New York NY.)

In order to express a biologically active HEPI, the nucleotide sequences encoding HEPI or derivatives thereof may be inserted into an appropriate expression vector, i.e., a vector which contains the necessary elements for transcriptional and translational control of the inserted coding sequence in a suitable host. These elements include regulatory sequences, such as enhancers, constitutive and inducible promoters, and 5' and 3' untranslated regions in the vector and in polynucleotide sequences encoding HEPI. Such elements may vary in their strength and specificity. Specific initiation signals may also be used to achieve more efficient translation of sequences encoding HEPI. Such signals include the ATG initiation codon and adjacent sequences, e.g. the Kozak sequence. In cases where sequences encoding HEPI and its initiation codon and upstream regulatory sequences are inserted into the appropriate expression vector, no additional transcriptional or translational control signals may be needed. However, in cases where only coding sequence, or a fragment thereof, is inserted, exogenous translational control signals including an in-frame ATG initiation codon should be provided by the vector. Exogenous translational elements and initiation codons may be of various origins, both natural and synthetic. The efficiency of expression may be enhanced by the inclusion of enhancers appropriate for the particular host cell system used. (See, e.g., Scharf, D. et al. (1994) Results Probl. Cell Differ. 20: 125-162.)

Methods which are well known to those skilled in the art may be used to construct expression vectors containing sequences encoding HEPI and appropriate transcriptional and translational control elements. These methods include in vitro recombinant DNA techniques, synthetic techniques, and in vivo genetic recombination. (See, e.g.,

Sambrook, J. et al. (1989) Molecular Cloning. A Laboratory Manual, Cold Spring Harbor Press, Plainview NY, ch. 4, 8, and 16-17; Ausubel, F.M. et al. (1995) Current Protocols in Molecular Biology, John Wiley & Sons, New York NY, ch. 9, 13, and 16.)

A variety of expression vector/host systems may be utilized to contain and express sequences encoding HEPI. These include, but are not limited to, microorganisms such as bacteria transformed with recombinant bacteriophage, plasmid, or cosmid DNA expression vectors; yeast transformed with yeast expression vectors; insect cell systems infected with viral expression vectors (e.g., baculovirus); plant cell systems transformed with viral expression vectors (e.g., cauliflower mosaic virus, CaMV, or tobacco mosaic virus, TMV) or with bacterial expression vectors (e.g., Ti or pBR322 plasmids); or animal cell systems. The invention is not limited by the host cell employed.

In bacterial systems, a number of cloning and expression vectors may be selected depending upon the use intended for polynucleotide sequences encoding HEPI. For example. routine cloning, subcloning, and propagation of polynucleotide sequences encoding HEPI can be achieved using a multifunctional E. coli vector such as PBLUESCRIPT (Stratagene, La Jolla CA) or pSPORT1 plasmid (Life Technologies). Ligation of sequences encoding HEPI into the vector's multiple cloning site disrupts the *lac*Z gene, allowing a colorimetric screening procedure for identification of transformed bacteria containing recombinant molecules. In addition, these vectors may be useful for in vitro transcription, dideoxy sequencing, single strand rescue with helper phage, and creation of nested deletions in the cloned sequence. (See, e.g., Van Heeke, G. and S.M. Schuster (1989) J. Biol. Chem. 264:5503-5509.) When large quantities of HEPI are needed, e.g. for the production of antibodies, vectors which direct high level expression of HEPI may be used. For example, vectors containing the strong, inducible T5 or T7 bacteriophage promoter may be used.

Yeast expression systems may be used for production of HEPI. A number of vectors containing constitutive or inducible promoters, such as alpha factor, alcohol oxidase, and PGH, may be used in the yeast Saccharomyces cerevisiae or Pichia pastoris. In addition, such vectors direct either the secretion or intracellular retention of expressed proteins and enable integration of foreign sequences into the host genome for stable propagation. (See, e.g., Ausubel, 1995, supra; Grant et al. (1987) Methods Enzymol. 153:516-54; and Scorer, C. A. et al. (1994) Bio/Technology 12: 181-184.)

Plant systems may also be used for expression of HEPI. Transcription of sequences encoding HEPI may be driven viral promoters, e.g., the 35S and 19S promoters of CaMV used alone or in combination with the omega leader sequence from TMV (Takamatsu, N. (1987) EMBO J. 6:307-311). Alternatively, plant promoters such as the small subunit of RUBISCO or heat shock promoters may be used. (See, e.g., Coruzzi, G. et al. (1984) EMBO J. 3:1671-1680; Broglie, R. et al. (1984) Science 224:838-843; and Winter, J. et al. (1991) Results Probl. Cell Differ. 17:85-105.) These constructs can be introduced into plant cells by direct DNA transformation or pathogen-mediated transfection. (See, e.g., The McGraw Hill Yearbook of Science and Technology (1992) McGraw Hill, New York NY, pp. 191-196.)

In mammalian cells, a number of viral-based expression systems may be utilized. In cases where an adenovirus is used as an expression vector, sequences encoding HEPI may be ligated into an adenovirus transcription/translation complex consisting of the late.promoter and tripartite leader sequence. Insertion in a non-essential E1 or E3 region of the viral genome may be used to obtain infective virus which expresses HEPI in host cells. (See, e.g., Logan, J. and T. Shenk (1984) Proc. Natl. Acad. Sci. 81:3655-3659.) In addition, transcription enhancers, such as the Rous sarcoma virus (RSV) enhancer, may be used to increase expression in mammalian host cells. SV40 or EBV-based vectors may also be used for high-level protein expression.

Human artificial chromosomes (HACs) may also be employed to deliver larger fragments of DNA than can be contained in and expressed from a plasmid. HACs of about 6 kb to 10 Mb are constructed and delivered via conventional delivery methods (liposomes, polycationic amino polymers, or vesicles) for therapeutic purposes. (See, e.g., Harrington, J.J. et al. (1997) Nat Genet. 15:345-355.)

For long term production of recombinant proteins in mammalian systems, stable expression of HEPI in cell lines is preferred. For example, sequences encoding HEPI can be transformed into cell lines using expression vectors which may contain viral origins of replication and/or endogenous expression elements and a selectable marker gene on the same or on a separate vector. Following the introduction of the vector, cells may be allowed to grow for about 1 to 2 days in enriched media before being switched to selective media. The purpose of the selectable marker is to confer resistance to a selective agent, and its presence allows growth and recovery of cells which successfully express the introduced sequences. Resistant clones of stably transformed cells may be propagated using tissue culture techniques appropriate to the cell type.

Any number of selection systems may be used to recover transformed cell lines. These include, but are not limited to, the herpes simplex virus thymidine kinase and adenine phosphoribosyltransferase genes, for use in $tk^-$ or $apr^-$ cells, respectively. (See, e.g., Wigler, M. et al. (1977) Cell 11:223-232; Lowy, I. et al. (1980) Cell 22:817-823.) Also, antimetabolite, antibiotic, or herbicide resistance can be used as the basis for selection. For example, *dhfr* confers resistance to methotrexate; *neo* confers resistance to the aminoglycosides, neomycin and G-418; and *als* or *pat* confer resistance to chlorsulfuron and phosphinotricin acetyltransferase, respectively. (See, e.g., Wigler, M. et al. (1980) Proc. Natl. Acad. Sci. 77:3567-3570; Colbere-Garapin, F. et al. (1981) J. Mol. Biol. 150:1-14.) Additional selectable genes have been described, e.g., *trpB* and *hisD,* which alter cellular requirements for metabolites. (See, e.g., Hartman, S.C. and R.C. Mulligan (1988) Proc. Natl. Acad. Sci. 85:8047-8051.) Visible markers, e.g., anthocyanins, green fluorescent proteins (GFP; Clontech), ß glucuronidase and its substrate β-glucuronide, or luciferase and its substrate luciferin may be used. These markers can be used not only to identify transformants, but also to quantify the amount of transient or stable protein expression attributable to a specific vector system. (See, e.g., Rhodes, C.A. (1995) Methods Mol. Biol. 55:121-131.)

Although the presence/absence of marker gene expression suggests that the gene of interest is also present, the presence and expression of the gene may need to be confirmed. For example, if the sequence encoding HEPI is inserted within a marker gene sequence, transformed cells containing sequences encoding HEPI can be identified by the absence of marker gene function. Alternatively, a marker gene can be placed in tandem with a sequence encoding HEPI under the control of a single promoter. Expression of the marker gene in response to induction or selection usually indicates expression of the tandem gene as well.

In general, host cells that contain the nucleic acid sequence encoding HEPI and that express HEPI may be identified by a variety of procedures known to those of skill in the art. These procedures include, but are not limited to, DNA-DNA or DNA-RNA hybridizations, PCR amplification, and protein bioassay or immunoassay techniques which include membrane, solution, or chip based technologies for the detection and/or quantification of nucleic acid or protein sequences.

Immunological methods for detecting and measuring the expression of HEPI using either specific polyclonal or monoclonal antibodies are known in the art. Examples of such techniques include enzyme-linked immunosorbent assays (ELISAs), radioimmunoassays (RIAs), and fluorescence activated cell sorting (FACS). A two-site, monoclonal-based immunoassay utilizing monoclonal antibodies reactive to two non-interfering epitopes on HEPI is preferred, but a competitive binding assay may be employed. These and other assays are well known in the art. (See, e.g., Hampton, R. et al. (1990) Serological Methods, a Laboratory Manual, APS Press, St Paul MN, Sect. IV; Coligan, J. E. et al. (1997) Current Protocols in Immunology, Greene Pub. Associates and Wiley-Interscience, New York NY; and Pound, J.D. (1998) Immunochemical Protocols, Humana Press, Totowa NJ).

A wide variety of labels and conjugation techniques are known by those skilled in the art and may be used in various nucleic acid and amino acid assays. Means for producing labeled hybridization or PCR probes for detecting sequences related to polynucleotides encoding HEPI include oligolabeling, nick translation, end-labeling, or PCR amplification using a labeled nucleotide. Alternatively, the sequences encoding HEPI, or any fragments thereof, may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits, such as those provided by Amersham Pharmacia Biotech, Promega (Madison WI), and US Biochemical. Suitable reporter molecules or labels which may be used for ease of detection include radionuclides, enzymes, fluorescent, chemiluminescent, or chromogenic agents, as well as substrates, cofactors, inhibitors, magnetic particles, and the like.

Host cells transformed with nucleotide sequences encoding HEPI may be cultured under conditions suitable for the expression and recovery of the protein from cell culture. The protein produced by a transformed cell may be secreted or retained intracellularly depending on the sequence and/or the vector used. As will be understood by those of skill in the art, expression vectors containing polynucleotides which encode HEPI may be designed to contain signal sequences which direct secretion of HEPI through a prokaryotic or eukaryotic cell membrane.

In addition, a host cell strain may be chosen for its ability to modulate expression of the inserted sequences or to process the expressed protein in the desired fashion. Such modifications of the polypeptide include, but are not limited to, acetylation, carboxylation, glycosylation, phosphorylation, lipidation, and acylation. Post-translational processing which cleaves a "prepro" form of the protein may also be used to specify protein targeting, folding, and/or activity. Different host cells which have specific cellular machinery and characteristic mechanisms for post-translational activities (e.g., CHO, HeLa, MDCK, HEK293, and WI38), are available from the American Type Culture Collection (ATCC, Manassas, VA) and may be chosen to ensure the correct modification and processing of the foreign protein.

In another embodiment of the invention, natural, modified, or recombinant nucleic acid sequences encoding HEPI may be ligated to a heterologous sequence resulting in translation of a fusion protein in any of the aforementioned host systems. For example, a chimeric HEPI protein containing a heterologous moiety that can be recognized by a commercially available antibody may facilitate the screening of peptide libraries for inhibitors of HEPI activity. Heterologous protein and peptide moieties may also facilitate purification of fusion proteins using commercially available affinity matrices. Such moieties include, but are not limited to, glutathione S-transferase (GST), maltose binding protein (MBP), thioredoxin (Trx), calmodulin binding peptide (CBP), 6-His, FLAG, *c-myc,* and hemagglutinin (HA). GST, MBP, Trx, CBP, and 6-His enable purification of their cognate fusion proteins on immobilized glutathione, maltose, phenylarsine oxide, calmodulin, and metal-chelate resins, respectively. FLAG, *c-myc,* and hemagglutinin (HA) enable immunoaffinity purification of fusion proteins using commercially available monoclonal and polyclonal antibodies that specifically recognize these epitope tags. A fusion protein may also be engineered to contain a proteolytic cleavage site located between the HEPI encoding sequence and the heterologous protein sequence, so that HEPI may be cleaved away from the heterologous moiety following purification. Methods for fusion protein expression and purification are discussed in Ausubel (1995, supra, ch 10). A variety of commercially available kits may also be used to facilitate expression and purification of fusion proteins.

In a further embodiment of the invention, synthesis of radiolabeled HEPI may be achieved in vitro using the TNT rabbit reticulocyte lysate or wheat germ extract systems (Promega). These systems couple transcription and translation of protein-coding sequences operably associated with the T7, T3, or SP6 promoters. Translation takes place in the presence of a radiolabeled amino acid precursor, preferably $^{35}S$-methionine.

Fragments of HEPI may be produced not only by recombinant production, but also by direct peptide synthesis using solid-phase techniques. (See, e.g., Creighton, supra. pp. 55-60.) Protein synthesis may be performed by manual techniques or by automation. Automated synthesis may be achieved, for example, using the ABI 431A Peptide Synthesizer (Perkin-Elmer). Various fragments of HEPI may be synthesized separately and then combined to produce the full length molecule.

## THERAPEUTICS

Chemical and structural similarity, e.g., in the context of sequences and motifs, exists between regions of HEPI and human epidermal proteins. In addition, the expression of HEPI is closely associated with cell proliferation, cancer, inflammation, and immune response. Therefore, HEPI appears to play a role in epithelial, cell proliferative, and autoimmune/inflammatory disorders. In the treatment of disorders associated with increased HEPI expression or activity, it is desirable to decrease the expression or activity of HEPI. In the treatment of the above conditions associated with decreased HEPI expression or activity, it is desirable to increase the expression or activity of HEPI.

Therefore, in one embodiment, HEPI or a fragment or derivative thereof may be administered to a subject to treat or prevent a disorder associated with decreased expression or activity of HEPI. Examples of such disorders include, but are not limited to, an epithelial disorder, such as dyshidrotic eczema, allergic contact dermatitis, keratosis pilaris, melasma, vitiligo, actinic keratosis, basal cell carcinoma, squamous cell carcinoma, seborrheic keratosis, folliculitis, herpes simplex, herpes zoster, varicella, candidiasis, dermatophytosis, scabies, insect bites, cherry angioma, keloid, dermatofibroma, acrochordons, urticaria, transient acantholytic dermatosis, xerosis, eczema, atopic dermatitis, contact dermatitis, hand eczema, nummular eczema, lichen simplex chronicus, asteatotic eczema, stasis dermatitis and stasis ulceration, seborrheic dermatitis, psoriasis, lichen planus, pityriasis rosea, impetigo, ecthyma, dermatophytosis, tinea versicolor, warts, acne vulgaris, acne rosacea, pemphigus vulgaris, pemphigus foliaceus, paraneoplastic pemphigus, bullous pemphigoid, herpes gestationis, dermatitis herpetiformis, linear IgA disease, epidermolysis bullosa acquisita, dermatomyositis, lupus erythematosus, scleroderma and morphea, erythroderma, alopecia, figurate skin lesions, telangiectasias, hypopigmentation. hyperpigmentation, vesicles/bullae, exanthems, cutaneous drug reactions, papulonodular skin lesions, photosensitivity diseases, epidermolysis bullosa simplex, epidermolytic hyperkeratosis, epidermolytic and nonepidermolytic palmoplantar keratoderma, ichthyosis bullosa of Siemens. ichthyosis exfoliativa, keratosis palmaris et plantaris, keratosis palmoplantaris, palmoplantar keratoderma, keratosis punctata, Meesmann's corneal dystrophy, pachyonychia congenita. white sponge nevus, steatocystoma multiplex, epidermal nevi/epidermolytic hyperkeratosis type, monilethrix, trichothiodystrophy, chronic hepatitis/cryptogenic cirrhosis, and colorectal hyperplasia; a cell proliferative disorder, such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and, in particular, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus; and an autoimmune/inflammatory disorder, such as acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, autoimmune polyenodocrinopathy-candidiasis-ectodermal dystrophy (APECED), bronchitis, cholecystitis, contact dermatitis, Crohn's disease, atopic dermatitis, dermatomyositis, diabetes mellitus, emphysema, episodic lymphopenia with lymphocytotoxins, erythroblastosis fetalis, erythema nodosum, atrophic gastritis, glomerulonephritis, Goodpasture's syndrome, gout, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polymyositis, psoriasis, Reiter's syndrome, rheumatoid arthritis, scleroderma, Sjogren's syndrome, systemic anaphylaxis, systemic lupus erythematosus, systemic sclerosis, thrombocytopenic purpura, ulcerative colitis, uveitis, Werner syndrome, complications of cancer, hemodialysis, and extracorporeal circulation, viral, bacterial, fungal, parasitic, protozoal, and helminthic infections, and trauma.

In another embodiment, a vector capable of expressing HEPI or a fragment or derivative thereof may be administered to a subject to treat or prevent a disorder associated with decreased expression or activity of HEPI including, but not limited to, those described above.

In a further embodiment, a pharmaceutical composition comprising a substantially purified HEPI in conjunction with a suitable pharmaceutical carrier may be administered to a subject to treat or prevent a disorder associated with decreased expression or activity of HEPI including, but not limited to, those provided above.

In still another embodiment, an agonist which modulates the activity of HEPI may be administered to a subject to treat or prevent a disorder associated with decreased expression or activity of HEPI including, but not limited to, those listed above.

In a further embodiment, an antagonist of HEPI may be administered to a subject to treat or prevent a disorder associated with increased expression or activity of HEPI. Examples of such disorders include, but are not limited to, those epithelial, cell proliferative, and autoimmune/inflammatory disorders described above. In one aspect, an antibody which specifically binds HEPI may be used directly as an antagonist or indirectly as a targeting or delivery mechanism for bringing a pharmaceutical agent to cells or tissue which express HEPI.

In an additional embodiment, a vector expressing the complement of the polynucleotide encoding HEPI may be administered to a subject to treat or prevent a disorder associated with increased expression or activity of HEPI including, but not limited to, those described above.

In other embodiments, any of the proteins, antagonists, antibodies, agonists, complementary sequences, or vectors of the invention may be administered in combination with other appropriate therapeutic agents. Selection of the appropriate agents for use in combination therapy may be made by one of ordinary skill in the art, according to conventional pharmaceutical principles. The combination of therapeutic agents may act synergistically to effect the treatment or prevention of the various disorders described above. Using this approach, one may be able to achieve therapeutic efficacy with lower dosages of each agent, thus reducing the potential for adverse side effects.

An antagonist of HEPI may be produced using methods which are generally known in the art. In particular, purified HEPI may be used to produce antibodies or to screen libraries of pharmaceutical agents to identify those which specifically bind HEPI. Antibodies to HEPI may also be generated using methods that are well known in the art. Such antibodies may include, but are not limited to, polyclonal, monoclonal, chimeric, and single chain antibodies, Fab fragments, and fragments produced by a Fab expression library. Neutralizing antibodies (i.e., those which inhibit dimer formation) are especially preferred for therapeutic use.

For the production of antibodies, various hosts including goats, rabbits, rats, mice, humans, and others may be immunized by injection with HEPI or with any fragment or oligopeptide thereof which has immunogenic properties. Depending on the host species, various adjuvants may be used to increase immunological response. Such adjuvants include, but are not limited to, Freund's, mineral gels such as aluminum hydroxide, and surface active substances such as lysolecithin, pluronic polyols, polyanions, peptides, oil emulsions, KLH, and dinitrophenol. Among adjuvants used in humans, BCG (bacilli Calmette-Guerin) and Corynebacterium parvum are especially preferable.

It is preferred that the oligopeptides, peptides, or fragments used to induce antibodies to HEPI have an amino acid sequence consisting of at least about 5 amino acids, and, more preferably, of at least about 10 amino acids. It is also preferable that these oligopeptides, peptides, or fragments are identical to a portion of the amino acid sequence of the natural protein and contain the entire amino acid sequence of a small, naturally occurring molecule. Short stretches of HEPI amino acids may be fused with those of another protein, such as KLH, and antibodies to the chimeric molecule may be produced.

Monoclonal antibodies to HEPI may be prepared using any technique which provides for the production of antibody molecules by continuous cell lines in culture. These include, but are not limited to, the hybridoma technique, the human B-cell hybridoma technique, and the EBV-hybridoma technique. (See, e.g., Kohler, G. et al. (1975) Nature 256:495-497; Kozbor, D. et al. (1985) J. Immunol. Methods 81:31-42; Cote, R.J. et al. (1983) Proc. Natl. Acad. Sci. 80:2026-2030; and Cole, S.P. et al. (1984) Mol. Cell Biol. 62:109-120.)

In addition, techniques developed for the production of "chimeric antibodies," such as the splicing of mouse antibody genes to human antibody genes to obtain a molecule with appropriate antigen specificity and biological activity, can be used. (See, e.g., Morrison, S.L. et al. (1984) Proc. Natl. Acad. Sci. 81:6851-6855; Neuberger, M.S. et al. (1984) Nature 312:604-608; and Takeda, S. et al. (1985) Nature 314:452-454.) Alternatively, techniques described for the production of single chain antibodies may be adapted, using methods known in the art, to produce HEPI-specific single chain antibodies. Antibodies with related specificity, but of distinct idiotypic composition, may be generated by chain shuffling from random combinatorial immunoglobulin libraries. (See, e.g., Burton D.R. (1991) Proc. Natl. Acad. Sci. 88: 10134-10137.)

Antibodies may also be produced by inducing in vivo production in the lymphocyte population or by screening immunoglobulin libraries or panels of highly specific binding reagents as disclosed in the literature. (See, e.g., Orlandi, R. et al. (1989) Proc. Natl. Acad. Sci. 86: 3833-3837; Winter, G. et al. (1991) Nature 349:293-299.)

Antibody fragments which contain specific binding sites for HEPI may also be generated. For example, such fragments include, but are not limited to, F(ab')2 fragments produced by pepsin digestion of the antibody molecule and Fab fragments generated by reducing the disulfide bridges of the F(ab')2 fragments. Alternatively, Fab expression libraries may be constructed to allow rapid and easy identification of monoclonal Fab fragments with the desired specificity. (See, e.g., Huse, W.D. et al. (1989) Science 246:1275-1281.)

Various immunoassays may be used for screening to identify antibodies having the desired specificity. Numerous protocols for competitive binding or immunoradiometric assays using either polyclonal or monoclonal antibodies with established specificities are well known in the art. Such immunoassays typically involve the measurement of complex formation between HEPI and its specific antibody. A two-site, monoclonal-based immunoassay utilizing monoclonal antibodies reactive to two non-interfering HEPI epitopes is preferred, but a competitive binding assay may also be employed (Pound, supra).

Various methods such as Scatchard analysis in conjunction with radioimmunoassay techniques may be used to assess the affinity of antibodies for HEPI. Affinity is expressed as an association constant, $K_a$, which is defined as the molar concentration of HEPI-antibody complex divided by the molar concentrations of free antigen and free antibody under equilibrium conditions. The $K_a$ determined for a preparation of polyclonal antibodies, which are heterogeneous in their affinities for multiple HEPI epitopes, represents the average affinity, or avidity, of the antibodies for HEPI. The $K_a$ determined for a preparation of monoclonal antibodies, which are monospecific for a particular HEPI epitope, represents a true measure of affinity. High-affinity antibody preparations with K, ranging from about $10^9$ to $10^{12}$ L/mole are preferred for use in immunoassays in which the HEPI-antibody complex must withstand rigorous manipulations. Low-affinity antibody preparations with $K_a$ ranging from about $10^6$ to $10^7$ L/mole are preferred for use in immunopurification and similar procedures which ultimately require dissociation of HEPI, preferably in active form, from the antibody (Catty, D. (1988) Antibodies, Volume 1: A Practical Approach, IRL Press, Washington, DC; Liddell, J. E. and Cryer, A. (1991) A Practical Guide to Monoclonal Antibodies, John Wiley & Sons, New York NY).

The titer and avidity of polyclonal antibody preparations may be further evaluated to determine the quality and suitability of such preparations for certain downstream applications. For example, a polyclonal antibody preparation containing at least 1-2 mg specific antibody/ml, preferably 5-10 mg specific antibody/ml, is preferred for use in procedures requiring precipitation of HEPI-antibody complexes. Procedures for evaluating antibody specificity, titer, and avidity, and guidelines for antibody quality and usage in various applications, are generally available. (See, e.g., Catty, supra, and Coligan et al. supra.)

In another embodiment of the invention, the polynucleotides encoding HEPI, or any fragment or complement thereof, may be used for therapeutic purposes. In one aspect, the complement of the polynucleotide encoding HEPI may be used in situations in which it would be desirable to block the transcription of the mRNA. In particular, cells may be transformed with sequences complementary to polynucleotides encoding HEPI. Thus, complementary molecules or fragments may be used to modulate HEPI activity, or to achieve regulation of gene function. Such technology is now well known in the art, and sense or antisense oligonucleotides or larger fragments can be designed from various locations along the coding or control regions of sequences encoding HEPI.

Expression vectors derived from retroviruses, adenoviruses. or herpes or vaccinia viruses, or from various bacterial plasmids, may be used for delivery of nucleotide sequences to the targeted organ, tissue, or cell population. Methods which are well known to those skilled in the art can be used to construct vectors to express nucleic acid sequences complementary to the polynucleotides encoding HEPI. (See, e.g., Sambrook, supra; Ausubel, 1995, supra.)

Genes encoding HEPI can be turned off by transforming a cell or tissue with expression vectors which express high levels of a polynucleotide, or fragment thereof, encoding HEPI. Such constructs may be used to introduce untranslatable sense or antisense sequences into a cell. Even in the absence of integration into the DNA, such vectors may continue to transcribe RNA molecules until they are disabled by endogenous nucleases. Transient expression may last for a month or more with a non-replicating vector, and may last even longer if appropriate replication elements are part of the vector system.

As mentioned above, modifications of gene expression can be obtained by designing complementary sequences or antisense molecules (DNA, RNA, or PNA) to the control, 5', or regulatory regions of the gene encoding HEPI. Oligonucleotides derived from the transcription initiation site, e.g., between about positions -10 and +10 from the start site, are preferred. Similarly, inhibition can be achieved using triple helix base-pairing methodology. Triple helix pairing is useful because it causes inhibition of the ability of the double helix to open sufficiently for the binding of polymerases, transcription factors, or regulatory molecules. Recent therapeutic advances using triplex DNA have been described in the literature. (See, e.g., Gee, J.E. et al. (1994) in Huber, B.E. and B.I. Carr, Molecular and Immunologic Approaches, Futura Publishing, Mt. Kisco NY, pp. 163-177.) A complementary sequence or antisense molecule may also be designed to block translation of mRNA by preventing the transcript from binding to ribosomes.

Ribozymes, enzymatic RNA molecules, may also be used to catalyze the specific cleavage of RNA. The mechanism of ribozyme action involves sequence-specific hybridization of the ribozyme molecule to complementary target RNA, followed by endonucleolytic cleavage. For example, engineered hammerhead motif ribozyme molecules may specifically and efficiently catalyze endonucleolytic cleavage of sequences encoding HEPI.

Specific ribozyme cleavage sites within any potential RNA target are initially identified by scanning the target molecule for ribozyme cleavage sites, including the following sequences: GUA, GUU, and GUC. Once identified, short RNA sequences of between 15 and 20 ribonucleotides, corresponding to the region of the target gene containing the cleavage site, may be evaluated for secondary structural features which may render the oligonucleotide inoperable. The suitability of candidate targets may also be evaluated by testing accessibility to hybridization with complementary oligonucleotides using ribonuclease protection assays.

Complementary ribonucleic acid molecules and ribozymes of the invention may be prepared by any method known in the art for the synthesis of nucleic acid molecules. These include techniques for chemically synthesizing oligonucleotides such as solid phase phosphoramidite chemical synthesis. Alternatively, RNA molecules may be generated by in vitro and in vivo transcription of DNA sequences encoding HEPI. Such DNA sequences may be incorporated into a wide variety of vectors with suitable RNA polymerase promoters such as T7 or SP6. Alternatively, these cDNA constructs that synthesize complementary RNA, constitutively or inducibly, can be introduced into cell lines, cells, or tissues.

RNA molecules may be modified to increase intracellular stability and half-life. Possible modifications include, but are not limited to, the addition of flanking sequences at the 5' and/or 3' ends of the molecule, or the use of phosphorothioate or 2' O-methyl rather than phosphodiesterase linkages within the backbone of the molecule. This concept is inherent in the production of PNAs and can be extended in all of these molecules by the inclusion of nontraditional bases such as inosine, queosine, and wybutosine, as well as acetyl-, methyl-, thio-, and similarly modified forms of adenine, cytidine, guanine, thymine, and uridine which are not as easily recognized by endogenous endonucleases.

Many methods for introducing vectors into cells or tissues are available and equally suitable for use in vivo, in vitro, and ex vivo. For ex vivo therapy, vectors may be introduced into stem cells taken from the patient and clonally propagated for autologous transplant back into that same patient. Delivery by transfection, by liposome injections, or by polycationic amino polymers may be achieved using methods which are well known in the art. (See, e.g., Goldman, C.K. et al. (1997) Nature Biotechnology 15:462-466.)

Any of the therapeutic methods described above may be applied to any subject in need of such therapy, including, for example, mammals such as dogs, cats, cows, horses, rabbits, monkeys, and most preferably, humans.

An additional embodiment of the invention relates to the administration of a pharmaceutical or sterile composition, in conjunction with a pharmaceutically acceptable carrier, for any of the therapeutic effects discussed above. Such pharmaceutical compositions may consist of HEPI, antibodies to HEPI, and mimetics, agonists, antagonists, or inhibitors of HEPI. The compositions may be administered alone or in combination with at least one other agent, such as a stabilizing compound, which may be administered in any sterile, biocompatible pharmaceutical carrier including, but not limited to, saline, buffered saline, dextrose, and water. The compositions may be administered to a patient alone, or in combination with other agents, drugs, or hormones.

The pharmaceutical compositions utilized in this invention may be administered by any number of routes including, but not limited to, oral, intravenous, intramuscular, intra-arterial. intramedullary, intrathecal, intraventricular, transdermal, subcutaneous, intraperitoneal, intranasal, enteral, topical, sublingual, or rectal means.

In addition to the active ingredients, these pharmaceutical compositions may contain suitable pharmaceutically-acceptable carriers comprising excipients and auxiliaries which facilitate processing of the active compounds into preparations which can be used pharmaceutically. Further details on techniques for formulation and administration may be found in the latest edition of Remington's Pharmaceutical Sciences (Maack Publishing, Easton PA).

Pharmaceutical compositions for oral administration can be formulated using pharmaceutically acceptable carriers well known in the art in dosages suitable for oral administration. Such carriers enable the pharmaceutical compositions to be formulated as tablets, pills, dragees, capsules, liquids, gels, syrups, slurries, suspensions, and the like, for ingestion by the patient.

Pharmaceutical preparations for oral use can be obtained through combining active compounds with solid excipient and processing the resultant mixture of granules (optionally, after grinding) to obtain tablets or dragee cores. Suitable auxiliaries can be added, if desired. Suitable excipients include carbohydrate or protein fillers, such as sugars, including lactose, sucrose, mannitol, and sorbitol; starch from corn, wheat, rice, potato, or other plants; cellulose, such as methyl cellulose, hydroxypropylmethyl-cellulose, or sodium carboxymethylcellulose; gums, including arabic and tragacanth; and proteins, such as gelatin and collagen. If desired, disintegrating or solubilizing agents may be added, such as the cross-linked polyvinyl pyrrolidone, agar, and alginic acid or a salt thereof, such as sodium alginate.

Dragee cores may be used in conjunction with suitable coatings, such as concentrated sugar solutions, which may also contain gum arabic, talc, polyvinylpyrrolidone, carbopol gel, polyethylene glycol, and/or titanium dioxide, lacquer solutions, and suitable organic solvents or solvent mixtures. Dyestuffs or pigments may be added to the tablets or dragee coatings for product identification or to characterize the quantity of active compound, i.e., dosage.

Pharmaceutical preparations which can be used orally include push-fit capsules made of gelatin, as well as soft, sealed capsules made of gelatin and a coating, such as glycerol or sorbitol. Push-fit capsules can contain active ingredients mixed with fillers or binders, such as lactose or starches, lubricants, such as talc or magnesium stearate, and, optionally, stabilizers. In soft capsules, the active compounds may be dissolved or suspended in suitable liquids, such as fatty oils, liquid, or liquid polyethylene glycol with or without stabilizers.

Pharmaceutical formulations suitable for parenteral administration may be formulated in aqueous solutions, preferably in physiologically compatible buffers such as Hanks' solution. Ringer's solution, or physiologically buffered saline. Aqueous injection suspensions may contain substances which increase the viscosity of the suspension, such as sodium carboxymethyl cellulose, sorbitol, or dextran. Additionally, suspensions of the active compounds may be prepared as appropriate oily injection suspensions. Suitable lipophilic solvents or vehicles include fatty oils, such as sesame oil, or synthetic fatty acid esters, such as ethyl oleate, triglycerides, or liposomes. Non-lipid polycationic amino polymers may also be used for delivery. Optionally, the suspension may also contain suitable stabilizers or agents to increase the solubility of the compounds and allow for the preparation of highly concentrated solutions.

For topical or nasal administration, penetrants appropriate to the particular barrier to be permeated are used in the formulation. Such penetrants are generally known in the art.

The pharmaceutical compositions of the present invention may be manufactured in a manner that is known in the art, e.g., by means of conventional mixing, dissolving, granulating, dragee-making, levigating, emulsifying, encapsulating, entrapping, or lyophilizing processes.

The pharmaceutical composition may be provided as a salt and can be formed with many acids, including but not limited to, hydrochloric, sulfuric, acetic, lactic, tartaric, malic, and succinic acid. Salts tend to be more soluble in aqueous or other protonic solvents than are the corresponding free base forms. In other cases, the preferred preparation may be a lyophilized powder which may contain any or all of the following: 1 mM to 50 mM histidine, 0.1% to 2% sucrose, and 2% to 7% mannitol, at a pH range of 4.5 to 5.5, that is combined with buffer prior to use.

After pharmaceutical compositions have been prepared, they can be placed in an appropriate container and labeled for treatment of an indicated condition. For administration of HEPI, such labeling would include amount, frequency, and method of administration.

Pharmaceutical compositions suitable for use in the invention include compositions wherein the active ingredients are contained in an effective amount to achieve the intended purpose. The determination of an effective dose is well within the capability of those skilled in the art.

For any compound, the therapeutically effective dose can be estimated initially either in cell culture assays, e.g., of neoplastic cells or in animal models such as mice, rats, rabbits, dogs, or pigs. An animal model may also be used to determine the appropriate concentration range and route of administration. Such information can then be used to determine useful doses and routes for administration in humans.

A therapeutically effective dose refers to that amount of active ingredient, for example HEPI or fragments thereof, antibodies of HEPI, and agonists, antagonists or inhibitors of HEPI, which ameliorates the symptoms or condition. Therapeutic efficacy and toxicity may be determined by standard pharmaceutical procedures in cell cultures or with experimental animals, such as by calculating the $ED_{50}$ (the dose therapeutically effective in 50% of the population) or $LD_{50}$ (the dose lethal to 50% of the population) statistics. The dose ratio of toxic to therapeutic effects is the therapeutic index, and it can be expressed as the $LD_{50}/ED_{50}$ ratio. Pharmaceutical compositions which exhibit large therapeutic indices are preferred. The data obtained from cell culture assays and animal studies are used to formulate a range of dosage for human use. The dosage contained in such compositions is preferably within a range of circulating concentrations that includes the $ED_{50}$ with little or no toxicity. The dosage varies within this range depending upon the dosage form employed, the sensitivity of the patient, and the route of administration.

The exact dosage will be determined by the practitioner, in light of factors related to the subject requiring treatment. Dosage and administration are adjusted to provide sufficient levels of the active moiety or to maintain the desired effect. Factors which may be taken into account include the severity of the disease state, the general health of the subject, the age, weight, and gender of the subject, time and frequency of administration, drug combination(s), reaction sensitivities, and response to therapy. Long-acting pharmaceutical compositions may be administered every 3 to 4 days, every week, or biweekly depending on the half-life and clearance rate of the particular formulation.

Normal dosage amounts may vary from about 0.1 μg to 100,000 μg, up to a total dose of about 1 gram, depending upon the route of administration. Guidance as to particular dosages and methods of delivery is provided in the literature and generally available to practitioners in the art. Those skilled in the art will employ different formulations for nucleotides than for proteins or their inhibitors. Similarly, delivery of polynucleotides or polypeptides will be specific to particular cells, conditions, locations, etc.

## DIAGNOSTICS

In another embodiment, antibodies which specifically bind HEPI may be used for the diagnosis of disorders characterized by expression of HEPI, or in assays to monitor patients being treated with HEPI or agonists, antagonists, or inhibitors of HEPI. Antibodies useful for diagnostic purposes may be prepared in the same manner as described above for therapeutics. Diagnostic assays for HEPI include methods which utilize the antibody and a label to detect HEPI in human body fluids or in extracts of cells or tissues. The antibodies may be used with or without modification, and may be labeled by covalent or non-covalent attachment of a reporter molecule. A wide variety of reporter molecules.

several of which are described above, are known in the art and may be used.

A variety of protocols for measuring HEPI, including ELISAs, RIAs, and FACS, are known in the art and provide a basis for diagnosing altered or abnormal levels of HEPI expression. Normal or standard values for HEPI expression are established by combining body fluids or cell extracts taken from normal mammalian subjects, preferably human, with antibody to HEPI under conditions suitable for complex formation. The amount of standard complex formation may be quantitated by various methods, preferably by photometric means. Quantities of HEPI expressed in subject, control, and disease samples from biopsied tissues are compared with the standard values. Deviation between standard and subject values establishes the parameters for diagnosing disease.

In another embodiment of the invention, the polynucleotides encoding HEPI may be used for diagnostic purposes. The polynucleotides which may be used include oligonucleotide sequences, complementary RNA and DNA molecules, and PNAs. The polynucleotides may be used to detect and quantitate gene expression in biopsied tissues in which expression of HEPI may be correlated with disease. The diagnostic assay may be used to determine absence, presence, and excess expression of HEPI, and to monitor regulation of HEPI levels during therapeutic intervention.

In one aspect, hybridization with PCR probes which are capable of detecting polynucleotide sequences, including genomic sequences, encoding HEPI or closely related molecules may be used to identify nucleic acid sequences which encode HEPI. The specificity of the probe, whether it is made from a highly specific region, e.g., the 5' regulatory region, or from a less specific region, e.g., a conserved motif, and the stringency of the hybridization or amplification (maximal, high, intermediate, or low), will determine whether the probe identifies only naturally occurring sequences encoding HEPI, allelic variants, or related sequences.

Probes may also be used for the detection of related sequences, and should preferably have at least 50% sequence identity to any of the HEPI encoding sequences. The hybridization probes of the subject invention may be DNA or RNA and may be derived from the sequence of SEQ ID NO:7-12 or from genomic sequences including promoters, enhancers, and introns of the HEPI gene.

Means for producing specific hybridization probes for DNAs encoding HEPI include the cloning of polynucleotide sequences encoding HEPI or HEPI derivatives into vectors for the production of mRNA probes. Such vectors are known in the art, are commercially available, and may be used to synthesize RNA probes in vitro by means of the addition of the appropriate RNA polymerases and the appropriate labeled nucleotides. Hybridization probes may be labeled by a variety of reporter groups, for example, by radionuclides such as $^{32}P$ or $^{35}S$, or by enzymatic labels, such as alkaline phosphatase coupled to the probe via avidin/biotin coupling systems, and the like.

Polynucleotide sequences encoding HEPI may be used for the diagnosis of disorders associated with expression of HEPI. Examples of such disorders include, but are not limited to, an epithelial disorder, such as dyshidrotic eczema, allergic contact dermatitis, keratosis pilaris, melasma, vitiligo, actinic keratosis, basal cell carcinoma, squamous cell carcinoma, seborrheic keratosis, folliculitis, herpes simplex, herpes zoster, varicella, candidiasis, dermatophytosis, scabies, insect bites, cherry angioma, keloid, dermatofibroma, acrochordons, urticaria, transient acantholytic dermatosis, xerosis, eczema, atopic dermatitis, contact dermatitis, hand eczema, nummular eczema, lichen simplex chronicus, asteatotic eczema, stasis dermatitis and stasis ulceration, seborrheic dermatitis, psoriasis, lichen planus, pityriasis rosea, impetigo, ecthyma, dermatophytosis, tinea versicolor, warts, acne vulgaris, acne rosacea, pemphigus vulgaris, pemphigus foliaceus, paraneoplastic pemphigus, bullous pemphigoid, herpes gestationis, dermatitis herpetiformis, linear IgA disease, epidermolysis bullosa acquisita, dermatomyositis, lupus erythematosus, scleroderma and morphea, erythroderma, alopecia, figurate skin lesions, telangiectasias, hypopigmentation, hyperpigmentation, vesicles/bullae, exanthems, cutaneous drug reactions, papulonodular skin lesions, photosensitivity diseases, epidermolysis bullosa simplex, epidermolytic hyperkeratosis, epidermolytic and nonepidermolytic palmoplantar keratoderma, ichthyosis bullosa of Siemens, ichthyosis exfoliativa, keratosis palmaris et plantaris, keratosis palmoplantaris, palmoplantar keratoderma, keratosis punctata, Meesmann's corneal dystrophy, pachyonychia congenita, white sponge nevus, steatocystoma multiplex, epidermal nevi/epidermolytic hyperkeratosis type, monilethrix, trichothiodystrophy, chronic hepatitis/cryptogenic cirrhosis, and colorectal hyperplasia; a cell proliferative disorder, such as actinic keratosis, arteriosclerosis, atherosclerosis, bursitis, cirrhosis, hepatitis, mixed connective tissue disease (MCTD), myelofibrosis, paroxysmal nocturnal hemoglobinuria, polycythemia vera, psoriasis, primary thrombocythemia, and cancers including adenocarcinoma, leukemia, lymphoma, melanoma, myeloma, sarcoma, teratocarcinoma, and, in particular, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus; and an autoimmune/inflammatory disorder, such as acquired immunodeficiency syndrome (AIDS), Addison's disease, adult respiratory distress syndrome, allergies, ankylosing spondylitis, amyloidosis, anemia, asthma, atherosclerosis, autoimmune hemolytic anemia, autoimmune thyroiditis, autoimmune polyenodocrinopathy-candidiasis-ectodermal dystrophy (APECED), bronchitis, cholecystitis, contact dermatitis, Crohn's disease, atopic dermatitis, dermatomyositis. diabetes mellitus, emphysema, episodic lymphopenia with lymphocytotoxins, erythroblastosis fetalis, erythema nodosum, atrophic gastritis, glomerulonephritis, Goodpasture's syndrome, gout, Graves' disease, Hashimoto's thyroiditis, hypereosinophilia, irritable bowel syndrome, multiple sclerosis, myasthenia gravis, myocardial or pericardial inflammation, osteoarthritis, osteoporosis, pancreatitis, polymyositis, psoriasis, Reiter's syndrome, rheumatoid arthritis, scleroderma, Sjogren's syndrome, systemic anaphylaxis, systemic lupus erythematosus, systemic sclerosis, thrombocytopenic purpura, ulcerative colitis, uveitis, Werner syndrome, complications of cancer, hemodialysis, and extracorporeal circulation, viral, bacterial, fungal, parasitic, protozoal, and helminthic infections, and trauma. The polynucleotide sequences encoding HEPI may be used in Southern or northern analysis, dot blot, or other membrane-based technologies; in PCR technologies; in dipstick, pin, and multiformat ELISA-like assays; and in microarrays utilizing fluids or tissues from patients to detect altered HEPI expression. Such qualitative or quantitative methods are well known in the art.

In a particular aspect, the nucleotide sequences encoding HEPI may be useful in assays that detect the presence of associated disorders, particularly those mentioned above. The nucleotide sequences encoding HEPI may be labeled by standard methods and added to a fluid or tissue sample from a patient under conditions suitable for the formation of hybridization complexes. After a suitable incubation period, the sample is washed and the signal is quantitated and compared with a standard value. If the amount of signal in the patient sample is significantly altered in comparison to a control sample then the presence of altered levels of nucleotide sequences encoding HEPI in the sample indicates the presence of the associated disorder. Such assays may also be used to evaluate the efficacy of a particular therapeutic treatment regimen in animal studies, in clinical trials, or to monitor the treatment of an individual patient.

In order to provide a basis for the diagnosis of a disorder associated with expression of HEPI, a normal or standard profile for expression is established. This may be accomplished by combining body fluids or cell extracts taken from normal subjects, either animal or human, with a sequence, or a fragment thereof, encoding HEPI, under conditions suitable for hybridization or amplification. Standard hybridization may be quantified by comparing the values obtained from normal subjects with values from an experiment in which a known amount of a substantially purified polynucleotide is used. Standard values obtained in this manner may be compared with values obtained from samples from patients who are symptomatic for a disorder. Deviation from standard values is used to establish the presence of a disorder.

Once the presence of a disorder is established and a treatment protocol is initiated, hybridization assays may be repeated on a regular basis to determine if the level of expression in the patient begins to approximate that which is observed in the normal subject. The results obtained from successive assays may be used to show the efficacy of treatment over a period ranging from several days to months.

With respect to cancer, the presence of an abnormal amount of transcript (either under- or overexpressed) in biopsied tissue from an individual may indicate a predisposition for the development of the disease, or may provide a means for detecting the disease prior to the appearance of actual clinical symptoms. A more definitive diagnosis of this type may allow health professionals to employ preventative measures or aggressive treatment earlier thereby preventing the development or further progression of the cancer.

Additional diagnostic uses for oligonucleotides designed from the sequences encoding HEPI may involve the use of PCR. These oligomers may be chemically synthesized, generated enzymatically, or produced in vitro. Oligomers will preferably contain a fragment of a polynucleotide encoding HEPI, or a fragment of a polynucleotide complementary to the polynucleotide encoding HEPI, and will be employed under optimized conditions for identification of a specific gene or condition. Oligomers may also be employed under less stringent conditions for detection or quantitation of closely related DNA or RNA sequences.

Methods which may also be used to quantify the expression of HEPI include radiolabeling or biotinylating nucleotides, coamplification of a control nucleic acid, and interpolating results from standard curves. (See, e.g., Melby, P.C. et al. (1993) J. Immunol. Methods 159:235-244; Duplaa, C. et al. (1993) Anal. Biochem. 212:229-236.) The speed of quantitation of multiple samples may be accelerated by running the assay in an ELISA format where the oligomer of interest is presented in various dilutions and a spectrophotometric or colorimetric response gives rapid quantitation.

In further embodiments, oligonucleotides or longer fragments derived from any of the polynucleotide sequences described herein may be used as targets in a microarray. The microarray can be used to monitor the expression level of large numbers of genes simultaneously and to identify genetic variants, mutations, and polymorphisms. This information may be used to determine gene function, to understand the genetic basis of a disorder, to diagnose a disorder, and to develop and monitor the activities of therapeutic agents.

Microarrays may be prepared, used, and analyzed using methods known in the art. (See, e.g., Brennan, T. M. et al. (1995) U.S. Patent No. 5,474,796; Schena, M. et al. (1996) Proc. Natl. Acad. Sci. 93:10614-10619; Baldeschweiler et al. (1995) PCT application WO95/251116; Shalon, D. et al. (1995) PCT application WO95/35505; Heller, R.A. et al. (1997) Proc. Natl. Acad. Sci. 94:2150-2155; and Heller, M.J. et al. (1997) U.S. Patent No. 5,605,662.)

In another embodiment of the invention, nucleic acid sequences encoding HEPI may be used to generate hybridization probes useful in mapping the naturally occurring genomic sequence. The sequences may be mapped to a particular chromosome, to a specific region of a chromosome, or to artificial chromosome constructions, e.g., human artificial chromosomes (HACs), yeast artificial chromosomes (YACs), bacterial artificial chromosomes (BACs), bacterial PI constructions, or single chromosome cDNA libraries. (See, e.g., Harrington, J.J. et al. (1997) Nat Genet. 15:345-355; Price, C.M. (1993) Blood Rev. 7:127-134; and Trask, B.J. (1991) Trends Genet. 7:149-154.)

Fluorescent in situ hybridization (FISH) may be correlated with other physical chromosome mapping techniques and genetic map data. (See, e.g., Heinz-Ulrich, et al. (1995) in Meyers, supra, pp. 965-968.) Examples of genetic map data can be found in various scientific journals or at the Online Mendelian Inheritance in Man (OMIM) site. Correlation between the location of the gene encoding HEPI on a physical chromosomal map and a specific disorder, or a predisposition to a specific disorder, may help define the region of DNA associated with that disorder. The nucleotide sequences of the invention may be used to detect differences in gene sequences among normal, carrier, and affected individuals.

In situ hybridization of chromosomal preparations and physical mapping techniques, such as linkage analysis using established chromosomal markers, may be used for extending genetic maps. Often the placement of a gene on the chromosome of another mammalian species, such as mouse, may reveal associated markers even if the number or arm of a particular human chromosome is not known. New sequences can be assigned to chromosomal arms by physical mapping. This provides valuable information to investigators searching for disease genes using positional cloning or other gene discovery techniques. Once the disease or syndrome has been crudely localized by genetic linkage to a particular genomic region, e.g., ataxia-telangiectasia to 11q22-23, any sequences mapping to that area may represent associated or regulatory genes for further investigation. (See, e.g., Gatti, R.A. et al. (1988) Nature 336: 577-580.) The nucleotide sequence of the subject invention may also be used to detect differences in the chromosomal location due to translocation, inversion, etc., among normal, carrier, or affected individuals.

In another embodiment of the invention, HEPI, its catalytic or immunogenic fragments, or oligopeptides thereof can be used for screening libraries of compounds in any of a variety of drug screening techniques. The fragment employed in such screening may be free in solution, affixed to a solid support, borne on a cell surface, or located intracellularly. The formation of binding complexes between HEPI and the agent being tested may be measured.

Another technique for drug screening provides for high throughput screening of compounds having suitable binding affinity to the protein of interest. (See, e.g., Geysen, et al. (1984) PCT application WO84/03564.) In this method, large numbers of different small test compounds are synthesized on a solid substrate. The test compounds are reacted with HEPI, or fragments thereof, and washed. Bound HEPI is then detected by methods well known in the art. Purified HEPI can also be coated directly onto plates for use in the aforementioned drug screening techniques. Alternatively, non-neutralizing antibodies can be used to capture the peptide and immobilize it on a solid support.

In another embodiment, one may use competitive drug screening assays in which neutralizing antibodies capable of binding HEPI specifically compete with a test compound for binding HEPI. In this manner, antibodies can be used to detect the presence of any peptide which shares one or more antigenic determinants with HEPI.

In additional embodiments, the nucleotide sequences which encode HEPI may be used in any molecular biology techniques that have yet to be developed, provided the new techniques rely on properties of nucleotide sequences that are currently known, including, but not limited to, such properties as the triplet genetic code and specific base pair interactions.

Without further elaboration, it is believed that one skilled in the art can, using the preceding description, utilize the present invention to its fullest extent. The following preferred specific embodiments are, therefore, to be construed as merely illustrative, and not limitative of the remainder of the disclosure in any way whatsoever.

The disclosures of all patents, applications, and publications mentioned above and below, in particular U.S. Ser. No. [Atty Docket #PF-0567 P], filed July 28, 1998, and U.S. Ser. No. [Atty Docket #PF-0649 P], filed December 7, 1998, are hereby expressly incorporated by reference.

## EXAMPLES

### I. Construction of cDNA Libraries

RNA was purchased from Clontech or isolated from tissues described in Table 4. Some tissues were homogenized and lysed in guanidinium isothiocyanatc, while others were homogenized and lysed in phenol or in a suitable mixture of denaturants, such as TRIZOL (Life Technologies), a monophasic solution of phenol and guanidine isothiocyanate. The resulting lysates were centrifuged over CsCl cushions or extracted with chloroform. RNA was precipitated from the lysates with either isopropanol or sodium acetate and ethanol, or by other routine methods.

Phenol extraction and precipitation of RNA were repeated as necessary to increase RNA purity. In some cases, RNA was treated with DNase. For most libraries, poly(A+) RNA was isolated using oligo d(T)-coupled paramagnetic particles (Promega), OLIGOTEX latex particles (QIAGEN, Chatsworth CA), or an OLIGOTEX mRNA purification kit (QIAGEN). Alternatively, RNA was isolated directly from tissue lysates using other RNA isolation kits, e.g., the POLY(A)PURE mRNA purification kit (Ambion, Austin TX).

In some cases, Stratagene was provided with RNA and constructed the corresponding cDNA libraries. Otherwise, cDNA was synthesized and cDNA libraries were constructed with the UNIZAP vector system (Stratagene) or SUPERSCRIPT plasmid system (Life Technologies), using the recommended procedures or similar methods known in the art. (See, e.g., Ausubel. 1997, supra, units 5.1-6.6). Reverse transcription was initiated using oligo d(T) or random primers. Synthetic oligonucleotide adapters were ligated to double stranded cDNA, and the cDNA was digested with the appropriate restriction enzyme or enzymes. For most libraries, the cDNA was size-selected (300-1000 bp) using SEPHACRYL S1000, SEPHAROSE CL2B, or SEPHAROSE CL4B column chromatography (Amersham Pharmacia Biotech) or preparative agarose gel electrophoresis. cDNAs were ligated into compatible restriction enzyme sites of the polylinker of a suitable plasmid, e.g., PBLUESCRIPT plasmid (Stratagene), pSPORT1 plasmid (Life Technologies), or pINCY (Incyte Pharmaceuticals, Palo Alto CA). Recombinant plasmids were transformed into competent E. coli cells including XL1-Blue, XL1-BlueMRF, or SOLR from Stratagene or DH5α, DH10B, or ElectroMAX DH10B from Life Technologies.

### II. Isolation of cDNA Clones

Plasmids were recovered from host cells by in vivo excision, using the UNIZAP vector system (Stratagene) or cell lysis. Plasmids were purified using at least one of the following: a Magic or WIZARD Minipreps DNA purification system (Promega); an AGTC Miniprep purification kit (Edge Biosystems, Gaithersburg MD); and QIAWELL 8 Plasmid, QIAWELL 8 Plus Plasmid, QIAWELL 8 Ultra Plasmid purification systems or the REAL Prep 96 plasmid kit from QIAGEN. Following precipitation, plasmids were resuspended in 0.1 ml of distilled water and stored, with or without lyophilization, at 4°C.

Alternatively, plasmid DNA was amplified from host cell lysates using direct link PCR in a high-throughput format (Rao, V.B. (1994) Anal. Biochem. 216:1-14). Host cell lysis and thermal cycling steps were carried out in a single reaction mixture. Samples were processed and stored in 384-well plates, and the concentration of amplified plasmid DNA was quantified fluorometrically using PICOGREEN dye (Molecular Probes, Eugene OR) and a Fluoroskan II fluorescence scanner (Labsystems Oy, Helsinki, Finland).

### III. Sequencing and Analysis

The cDNAs were prepared for sequencing using the ABI CATALYST 800 (Perkin-Elmer) or the HYDRA microdispenser (Robbins Scientific) or MICROLAB 2200 (Hamilton) systems in combination with the PTC-200 thermal cyclers (MJ Research). The cDNAs were sequenced using the ABI PRISM 373 or 377 sequencing systems (Perkin-Elmer) and standard ABI protocols, base calling software, and kits. In one alternative, cDNAs were sequenced using the MEGABACE 1000 DNA sequencing system (Molecular Dynamics). In another alternative, the cDNAs were amplified and sequenced using the ABI PRISM BIGDYE Terminator cycle sequencing ready reaction kit (Perkin-Elmer). In yet another alternative, cDNAs were sequenced using solutions and dyes from Amersham Pharmacia Biotech. Reading frames for the ESTs were determined using standard methods (reviewed in Ausubel, 1997, supra, unit 7.7). Some of the cDNA sequences were selected for extension using the techniques disclosed in Example V.

The polynucleotide sequences derived from cDNA, extension, and shotgun sequencing were assembled and analyzed using a combination of software programs which utilize algorithms well known to those skilled in the art. Table 5 summarizes the software programs, descriptions, references, and threshold parameters used. The first column of Table 5 shows the tools, programs, and algorithms used, the second column provides a brief description thereof, the third column presents the references which are incorporated by reference herein, and the fourth column presents, where applicable, the scores, probability values, and other parameters used to evaluate the strength of a match between two sequences (the higher the probability the greater the homology). Sequences were analyzed using MACDNASIS PRO software (Hitachi Software Engineering, South San Francisco CA) and LASERGENE software (DNASTAR).

The polynucleotide sequences were validated by removing vector, linker, and polyA sequences and by masking ambiguous bases, using algorithms and programs based on BLAST, dynamic programing, and dinucleotide nearest neighbor analysis. The sequences were then queried against a selection of public databases such as GenBank primate, rodent, mammalian, vertebrate, and eukaryote databases, and BLOCKS to acquire annotation, using programs based on BLAST, FASTA, and BLIMPS. The sequences were assembled into full length polynucleotide sequences using programs based on Phred, Phrap, and Consed, and were screened for open reading frames using programs based on GeneMark, BLAST, and PASTA. The full length polynucleotide sequences were translated to derive the corresponding full length amino acid sequences, and these full length sequences were subsequently analyzed by querying against databases such as the GenBank databases (described above), SwissProt, BLOCKS, PRINTS, Prosite, and Hidden Markov Model (HMM)-based protein family databases such as PFAM. HMM is a probabilistic approach which analyzes consensus primary structures of gene families. (See, e.g., Eddy, S.R. (1996) Curr. Opin. Str. Biol. 6:361-365.)

The programs described above for the assembly and analysis of full length polynucleotide and amino acid sequences were also used to identify polynucleotide sequence fragments from SEQ ID NO:7-12. Fragments from about 20 to about 4000 nucleotides which are useful in hybridization and amplification technologies were described in The Invention section above.

**IV. Northern Analysis**

Northern analysis is a laboratory technique used to detect the presence of a transcript of a gene and involves the hybridization of a labeled nucleotide sequence to a membrane on which RNAs from a particular cell type or tissue have been bound. (See, e.g., Sambrook, supra, ch. 7; Ausubel, 1995, supra, ch. 4 and 16.)

Analogous computer techniques applying BLAST were used to search for identical or related molecules in nucleotide databases such as GenBank or LIFESEQ database (Incyte Pharmaceuticals). This analysis is much faster than multiple membrane-based hybridizations. In addition, the sensitivity of the computer search can be modified to determine whether any particular match is categorized as exact or similar. The basis of the search is the product score, which is defined as:

$$\frac{\%\ \text{sequence identity x}\ \%\ \text{maximum BLAST score}}{100}$$

The product score takes into account both the degree of similarity between two sequences and the length of the sequence match. For example, with a product score of 40, the match will be exact within a 1% to 2% error, and, with a product score of 70, the match will be exact. Similar molecules are usually identified by selecting those which show product scores between 15 and 40, although lower scores may identify related molecules.

The results of northern analyses are reported as a percentage distribution of libraries in which the transcript encoding HEPI occurred. Analysis involved the categorization of cDNA libraries by organ/tissue and disease. The organ/tissue categories included cardiovascular, dermatologic, developmental, endocrine, gastrointestinal, hematopoietic/immune, musculoskeletal, nervous, reproductive, and urologic. The disease/condition categories included cancer, inflammation/trauma, cell proliferation, neurological, and pooled. For each category, the number of libraries expressing the sequence of interest was counted and divided by the total number of libraries across all categories.
Percentage values of tissue-specific and disease- or condition-specific expression are reported in the description of the invention.

**V. Extension of HEPI Encoding Polynucleotides**

The full length nucleic acid sequences of SEQ ID NO:7-9 were produced by extension of the component fragments described in Table 1, Column 5, using oligonucleotide primers based on those fragments. Primers were used to facilitate the extension of the known sequence "outward" generating amplicons containing new unknown nucleotide sequence for the region of interest. The initial primers were designed from the cDNA using OLIGO 4.06 software (National Biosciences, Plymouth, MN), or another appropriate program, to be about 22 to 30 nucleotides in length, to have a GC content of about 50% or more, and to anneal to the target sequence at temperatures of about 68°C to about 72°C. Any stretch of nucleotides which would result in hairpin structures and primer-primer dimerizations was avoided.

Selected human cDNA libraries (Life Technologies) were used to extend the sequence. If more than one extension is necessary or desired, additional sets of primers are designed to further extend the known region.

High fidelity amplification was obtained by following the instructions for the XL-PCR kit (The Perkin-Elmer Corp., Norwalk, CT) and thoroughly mixing the enzyme and reaction mix. PCR was performed using the PTC-200 thermal cycler (MJ Research, Inc., Watertown, MA), beginning with 40 pmol of each primer and the recommended concentrations of all other components of the kit, with the following parameters:

| | |
|---|---|
| Step 1 | 94° C for 1 min (initial denaturation) |
| Step 2 | 65° C for 1 min |
| Step 3 | 68° C for 6 min |
| Step 4 | 94° C for 15 sec |
| Step 5 | 65° C for 1 min |
| Step 6 | 68° C for 7 min |
| Step 7 | Repeat steps 4 through 6 for an additional 15 cycles |
| Step 8 | 94° C for 15 sec |
| Step 9 | 65° C for 1 min |
| Step 10 | 68° C for 7:15 min |

(continued)

| | |
|---|---|
| Step 11 | Repeat steps 8 through 10 for an additional 12 cycles |
| Step 12 | 72° C for 8 min |
| Step 13 | 4° C (and holding) |

A 5 µl to 10 µl aliquot of the reaction mixture was analyzed by electrophoresis on a low concentration (about 0.6% to 0.8%) agarose mini-gel to determine which reactions were successful in extending the sequence. Bands thought to contain the largest products were excised from the gel, purified using QIAQUICK purification kit (Qiagen, Inc.), and trimmed of overhangs using Klenow enzyme to facilitate religation and cloning.

After ethanol precipitation, the products were redissolved in 13 µ*l* of ligation buffer, 1 µ*l* T4-DNA ligase (15 units) and 1 µ*l* T4 polynucleotide kinase were added, and the mixture was incubated at room temperature for 2 to 3 hours, or overnight at 16° C. Competent E. coli cells (in 40 µ*l* of appropriate media) were transformed with 3 µ*l* of ligation mixture and cultured in 80 µl of SOC medium. (See, e.g., Sambrook, supra, Appendix A, p. 2.) After incubation for one hour at 37°C, the E. coli mixture was plated on Luria Bertani (LB) agar (See, e.g., Sambrook, supra, Appendix A, p. 1) containing carbenicillin (2x carb). The following day, several colonies were randomly picked from each plate and cultured in 150 µl of liquid LB/2x carb medium placed in an individual well of an appropriate commercially-available sterile 96-well microtiter plate. The following day, 5 µl of each overnight culture was transferred into a non-sterile 96-well plate and, after dilution 1:10 with water, 5 µl from each sample was transferred into a PCR array.

For PCR amplification, 18 µl of concentrated PCR reaction mix (3.3x) containing 4 units of rTth DNA polymerase, a vector primer, and one or both of the gene specific primers used for the extension reaction were added to each well. Amplification was performed using the following conditions:

| | |
|---|---|
| Step 1 | 94° C for 60 sec |
| Step 2 | 94° C for 20 sec |
| Step 3 | 55° C for 30 sec |
| Step 4 | 72° C for 90 sec |
| Step 5 | Repeat steps 2 through 4 for an additional 29 cycles |
| Step 6 | 72° C for 180 sec |
| Step 7 | 4° C (and holding) |

Aliquots of the PCR reactions were run on agarose gels together with molecular weight markers. The sizes of the PCR products were compared to the original partial cDNAs, and appropriate clones were selected, ligated into plasmid, and sequenced.

In like manner, the nucleotide sequences of SEQ ID NO:7-9 are used to obtain 5' regulatory sequences using the procedure above, oligonucleotides designed for 5' extension, and an appropriate genomic library.

The full length nucleic acid sequences of SEQ ID NO:10-12 were produced by extension of an appropriate fragment of the full length molecule using oligonucleotide primers designed from this fragment. One primer was synthesized to initiate 5' extension of the known fragment, and the other primer, to initiate 3' extension of the known fragment. The initial primers were designed using OLIGO 4.06 software (National Biosciences), or another appropriate program, to be about 22 to 30 nucleotides in length, to have a GC content of about 50% or more, and to anneal to the target sequence at temperatures of about 68°C to about 72°C. Any stretch of nucleotides which would result in hairpin structures and primer-primer dimerizations was avoided.

Selected human cDNA libraries were used to extend the sequence. If more than one extension was necessary or desired, additional or nested sets of primers were designed.

High fidelity amplification was obtained by PCR using methods well known in the art. PCR was performed in 96-well plates using the PTC-200 thermal cycler (MJ Research, Inc.). The reaction mix contained DNA template, 200 nmol of each primer, reaction buffer containing $Mg^{2+}$, $(NH_4)_2SO_4$, and β-mercaptoethanol, Taq DNA polymerase (Amersham Pharmacia Biotech), ELONGASE enzyme (Life Technologies), and Pfu DNA polymerase (Stratagene), with the following parameters for primer pair PCI A and PCI B: Step 1: 94°C, 3 min; Step 2: 94°C, 15 sec; Step 3: 60°C, 1 min; Step 4: 68°C, 2 min; Step 5: Steps 2, 3, and 4 repeated 20 times; Step 6: 68°C, 5 min; Step 7: storage at 4°C. In the alternative, the parameters for primer pair T7 and SK+ were as follows: Step 1: 94°C, 3 min; Step 2: 94°C, 15 sec; Step 3: 57°C, 1 min; Step 4: 68°C, 2 min; Step 5: Steps 2, 3, and 4 repeated 20 times; Step 6: 68°C, 5 min; Step 7: storage at 4°C.

The concentration of DNA in each well was determined by dispensing 100 µl PICOGREEN quantitation reagent (0.25% (v/v) PICOGREEN; Molecular Probes, Eugene OR) dissolved in 1X TE and 0.5 µl of undiluted PCR product into each well of an opaque fluorimeter plate (Coming Costar, Acton MA), allowing the DNA to bind to the reagent. The plate was scanned in a Fluoroskan II (Labsystems Oy, Helsinki, Finland) to measure the fluorescence of the sample and to quantify the concentration of DNA. A 5 μl to 10 μl aliquot of the reaction mixture was analyzed by electrophoresis on a 1 % agarose mini-gel to determine which reactions were successful in extending the sequence.

The extended nucleotides were desalted and concentrated, transferred to 384-well plates, digested with CviJI cholera virus endonuclease (Molecular Biology Research, Madison WI), and sonicated or sheared prior to religation into pUC 18 vector (Amersham Pharmacia Biotech). For shotgun sequencing, the digested nucleotides were separated on low concentration (0.6 to 0.8%) agarose gels, fragments were excised, and agar digested with Agar ACE (Promega). Extended clones were religated using T4 ligase (New England Biolabs, Beverly MA) into pUC 18 vector (Amersham Pharmacia Biotech), treated with Pfu DNA polymerase (Stratagene) to fill-in restriction site overhangs, and transfected into competent E. coli cells. Transformed cells were selected on antibiotic-containing media, individual colonies were picked and cultured overnight at 37°C in 384-well plates in LB/2x carb liquid media.

The cells were lysed, and DNA was amplified by PCR using Taq DNA polymerase (Amersham Pharmacia Biotech) and Pfu DNA polymerase (Stratagene) with the following parameters: Step 1: 94°C, 3 min; Step 2: 94°C, 15 sec; Step 3: 60°C, 1 min; Step 4: 72 °C, 2 min; Step 5: steps 2, 3, and 4 repeated 29 times; Step 6: 72°C, 5 min; Step 7: storage at 4°C. DNA was quantified by PICOGREEN reagent (Molecular Probes) as described above. Samples with low DNA recoveries were reamplified using the same conditions as described above. Samples were diluted with 20% dimethysulphoxide (1:2, v/v), and sequenced using DYENAMIC energy transfer sequencing primers and the DYENAMIC DIRECT kit (Amersham Pharmacia Biotech) or the ABI PRISM BIGDYE Terminator cycle sequencing ready reaction kit (Perkin-Elmer).

In like manner, the nucleotide sequences of SEQ ID NO:10-12 are used to obtain 5' regulatory sequences using the procedure above, oligonucleotides designed for such extension, and an appropriate genomic library.

### VI. Labeling and Use of Individual Hybridization Probes

Hybridization probes derived from SEQ ID NO:7-12 are employed to screen cDNAs, genomic DNAs, or mRNAs. Although the labeling of oligonucleotides, consisting of about 20 base pairs, is specifically described, essentially the same procedure is used with larger nucleotide fragments. Oligonucleotides are designed using state-of-the-art software such as OLIGO 4.06 software (National Biosciences) and labeled by combining 50 pmol of each oligomer, 250 μCi of [$\gamma$-$^{32}$P] adenosine triphosphate (Amersham Pharmacia Biotech), and T4 polynucleotide kinase (DuPont NEN, Boston MA). The labeled oligonucleotides are substantially purified using a SEPHADEX G-25 superfine size exclusion dextran bead column (Amersham Pharmacia Biotech). An aliquot containing $10^7$ counts per minute of the labeled probe is used in a typical membrane-based hybridization analysis of human genomic DNA digested with one of the following endonucleases: Ase I, Bgl II, Eco RI, Pst I, Xba1, or Pvu II (DuPont NEN).

The DNA from each digest is fractionated on a 0.7% agarose gel and transferred to nylon membranes (Nytran Plus, Schleicher & Schuell, Durham NH). Hybridization is carried out for 16 hours at 40°C. To remove nonspecific signals, blots are sequentially washed at room temperature under increasingly stringent conditions up to 0.1 x saline sodium citrate and 0.5% sodium dodecyl sulfate. After XOMAT-AR film (Eastman Kodak, Rochester NY) is exposed to the blots, hybridization patterns are compared visually.

### VII. Microarrays

A chemical coupling procedure and an ink jet device can be used to synthesize array elements on the surface of a substrate. (See, e.g., Baldeschweiler, supra.) An array analogous to a dot or slot blot may also be used to arrange and link elements to the surface of a substrate using thermal, UV, chemical, or mechanical bonding procedures. A typical array may be produced by hand or using available methods and machines and contain any appropriate number of elements. After hybridization, nonhybridized probes are removed and a scanner used to determine the levels and patterns of fluorescence. The degree of complementarity and the relative abundance of each probe which hybridizes to an element on the microarray may be assessed through analysis of the scanned images.

Full-length cDNAs, Expressed Sequence Tags (ESTs), or fragments thereof may comprise the elements of the microarray. Fragments suitable for hybridization can be selected using software well known in the art such as LASERGENE software (DNASTAR). Full-length cDNAs, ESTs, or fragments thereof corresponding to one of the nucleotide sequences of the present invention, or selected at random from a cDNA library relevant to the present invention, are arranged on an appropriate substrate, e.g., a glass slide. The cDNA is fixed to the slide using, e.g., UV crosslinking followed by thermal and chemical treatments and subsequent drying. (See, e.g., Schena, M. et al. (1995) Science 270:467-470; Shalon, D. et al. (1996) Genome Res. 6:639-645.) Fluorescent probes are prepared and used for hybridization to the elements on the substrate. The substrate is analyzed by procedures described above.

### VIII. Complementary Polynucleotides

Sequences complementary to the HEPI-encoding sequences, or any parts thereof, are used to detect, decrease, or inhibit expression of naturally occurring HEPI. Although use of oligonucleotides comprising from about 15 to 30 base pairs is described, essentially the same 5 procedure is used with smaller or with larger sequence fragments. Appropriate oligonucleotides are designed using OLIGO 4.06 software (National Biosciences) and the coding sequence of HEPI. To inhibit transcription, a complementary oligonucleotide is designed from the most unique 5' sequence and used to prevent promoter binding to the coding sequence. To inhibit translation, a complementary oligonucleotide is designed to prevent ribosomal binding to the HEPI-encoding transcript.

### IX. Expression of HEPI

Expression and purification of HEPI is achieved using bacterial or virus-based expression systems. For expression of HEPI in bacteria, cDNA is subcloned into an appropriate vector containing an antibiotic resistance gene and an inducible promoter that directs high levels of cDNA transcription. Examples of such promoters include, but are not limited to, the *trp-lac* (*tac*) hybrid promoter and the T5 or T7 bacteriophage promoter in conjunction with the *lac* operator regulatory element. Recombinant vectors are transformed into suitable bacterial hosts, e.g., BL21(DE3). Antibiotic resistant bacteria express HEPI upon induction with isopropyl beta-D-thiogalactopyranoside (IPTG). Expression of HEPI in eukaryotic cells is achieved by infecting insect or mammalian cell lines with recombinant Autographica californica nuclear polyhedrosis virus (AcMNPV), commonly known as baculovirus. The nonessential polyhedrin gene of baculovirus is replaced with cDNA encoding HEPI by either homologous recombination or bacterial-mediated transposition involving transfer plasmid intermediates. Viral infectivity is maintained and the strong polyhedrin promoter drives high levels of cDNA transcription. Recombinant baculovirus is used to infect Spodoptera frugiperda (Sf9) insect cells in most cases, or human hepatocytes, in some cases. Infection of the latter requires additional genetic modifications to baculovirus. (See Engelhard, E. K. et al. (1994) Proc. Natl. Acad. Sci. USA 91:3224-3227; Sandig, V. et al. (1996) Hum. Gene Ther. 7:1937-1945.)

In most expression systems, HEPI is synthesized as a fusion protein with, e.g., glutathione S-transferase (GST) or a peptide epitope tag, such as FLAG or 6-His, permitting rapid, single-step, affinity-based purification of recombinant fusion protein from crude cell lysates. GST, a 26-kilodalton enzyme from Schistosoma japonicum, enables the purification of fusion proteins on immobilized glutathione under conditions that maintain protein activity and antigenicity (Amersham Pharmacia Biotech). Following purification, the GST moiety can be proteolytically cleaved from HEPI at specifically engineered sites. FLAG, an 8-amino acid peptide, enables immunoaffinity purification using commercially available monoclonal and polyclonal anti-FLAG antibodies (Eastman Kodak). 6-His, a stretch of six consecutive histidine residues, enables purification on metal-chelate resins (QIAGEN). Methods for protein expression and purification are discussed in Ausubel (1995, supra, ch 10 and 16). Purified HEPI obtained by these methods can be used directly in the following activity assay.

### X. Demonstration of HEPI Activity

HEPI, or biologically active fragments thereof, are labeled with $^{125}I$ Bolton-Hunter reagent. (See, e.g., Bolton et al. (1973) Biochem. J. 133:529.) Candidate molecules previously arrayed in the wells of a multi-well plate are incubated with the labeled HEPI, washed, and any wells with labeled HEPI complex are assayed. Data obtained using different concentrations of HEPI are used to calculate values for the number, affinity, and association of HEPI with the candidate molecules.

### XI. Functional Assays

HEPI function is assessed by expressing the sequences encoding HEPI at physiologically elevated levels in mammalian cell culture systems. cDNA is subcloned into a mammalian expression vector containing a strong promoter that drives high levels of cDNA expression. Vectors of choice include pCMV SPORT (Life Technologies) and pCR3.1 (Invitrogen, Carlsbad CA), both of which contain the cytomegalovirus promoter. 5-10 μg of recombinant vector are transiently transfected into a human cell line, preferably of endothelial or hematopoietic origin, using either liposome formulations or electroporation. 1-2 μg of an additional plasmid containing sequences encoding a marker protein are co-transfected. Expression of a marker protein provides a means to distinguish transfected cells from nontransfected cells and is a reliable predictor of cDNA expression from the recombinant vector. Marker proteins of choice include, e. g., Green Fluorescent Protein (GFP; Clontech), CD64, or a CD64-GFP fusion protein. Flow cytometry (FCM), an automated, laser optics-based technique, is used to identify transfected cells expressing GFP or CD64-GFP and to evaluate properties, for example, their apoptotic state. FCM detects and quantifies the uptake of fluorescent molecules that diagnose events preceding or coincident with cell death. These events include changes in nuclear DNA content as measured by staining of DNA with propidium iodide; changes in cell size and granularity as measured by forward light scatter and 90 degree side light scatter; down-regulation of DNA synthesis as measured by decrease in bromodeoxyuridine uptake; alterations in expression of cell surface and intracellular proteins as measured by reactivity with specific antibodies; and alterations in plasma membrane composition as measured by the binding of fluorescein-conjugated Annexin V protein to the cell surface. Methods in flow cytometry are discussed in Ormerod, M. G. (1994) Flow Cytometry, Oxford, New York NY.

The influence of HEPI on gene expression can be assessed using highly purified populations of cells transfected with sequences encoding HEPI and either CD64 or CD64-GFP. CD64 and CD64-GFP are expressed on the surface of transfected cells and bind to conserved regions of human immunoglobulin G (IgG). Transfected cells are efficiently separated from nontransfected cells using magnetic beads coated with either human IgG or antibody against CD64 (DYNAL, Lake Success NY). mRNA can be purified from the cells using methods well known by those of skill in the art. Expression of mRNA encoding HEPI and other genes of interest can be analyzed by northern analysis or microarray techniques.

### XII. Production of HEPI Specific Antibodies

HEPI substantially purified using polyacrylamide gel electrophoresis (PAGE; see, e.g., Harrington, M.G. (1990) Methods Enzymol. 182:488-495), or other purification techniques, is used to immunize rabbits and to produce antibodies using standard protocols.

Alternatively, the HEPI amino acid sequence is analyzed using LASERGENE software (DNASTAR) to determine regions of high immunogenicity, and a corresponding oligopeptide is synthesized and used to raise antibodies by means known to those of skill in the art. Methods for selection of appropriate epitopes, such as those near the C-terminus or in hydrophilic regions are well described in the art. (See, e.g., Ausubel, 1995, supra, ch. 11.)

Typically, oligopeptides 15 residues in length are synthesized using an ABI 431 A Peptide Synthesizer (Perkin-Elmer) using fmoc-chemistry and coupled to KLH (Sigma-Aldrich, St. Louis MO) by reaction with N-maleimidobenzoyl-N-hydroxysuccinimide ester (MBS) to increase immunogenicity. (See, e.g., Ausubel, 1995, supra.) Rabbits are immunized with the oligopeptide-KLH complex in complete Freund's adjuvant. Resulting antisera are tested for antipeptide activity by, for example, binding the peptide to plastic, blocking with 1% BSA, reacting with rabbit antisera, washing, and reacting with radio-iodinated goat anti-rabbit IgG.

### XIII. Purification of Naturally Occurring HEPI Using Specific Antibodies

Naturally occurring or recombinant HEPI is substantially purified by immunoaffinity chromatography using antibodies specific for HEPI. An immunoaffinity column is constructed by covalently coupling anti-HEPI antibody to an activated chromatographic resin, such as CNBr-activated SEPHAROSE (Amersham Pharmacia Biotech). After the coupling, the resin is blocked and washed according to the manufacturer's instructions.

Media containing HEPI are passed over the immunoaffinity column, and the column is washed under conditions that allow the preferential absorbance of HEPI (e.g., high ionic strength buffers in the presence of detergent). The column is eluted under conditions that disrupt antibody/HEPI binding (e.g., a buffer of pH 2 to pH 3, or a high concentration of a chaotrope, such as urea or thiocyanate ion), and HEPI is collected.

Various modifications and variations of the described methods and systems of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

Table 1

| Polypeptide SEQ ID NO: | Nucleotide SEQ ID NO: | Clone ID | Library | Fragments |
|---|---|---|---|---|
| 1 | 7 | 2024646 | KERANOT02 | 2024646H1 (KERANOT02), 3098861H1 (CERVNOT03), 2024646T6 (KERANOT02) |
| 2 | 8 | 3431776 | SKINNOT04 | 3431776H1 (SKINNOT04), 3431776F6 (SKINNOT04), 3431776T6 (SKINNOT04) |
| 3 | 9 | 1798487 | COLNNOT27 | 1798487H1 (COLNNOT27), 3428602F6 (SKINNOT04), 3428602T6 (SKINNOT04) |
| 4 | 10 | 1448744 | PLACNOT02 | 1448744H1 (PLACNOT02), 2624313R6 (KERANOT02) |
| 5 | 11 | 2737275 | OVARNOT09 | 1232225H1 (LUNGFET03), 1305387T1 (PLACNOT02), 2179882X11F1 (SININOT01), 2737275H1 (OVARNOT09), 2798954F6 (NPOLNOT01), 4317843H1 (BRADDIT02) |
| 6 | 12 | 3325323 | PTHYNOT03 | 3325323H1 (PTHYNOT03), 3517325R6 (LUNGNON03), SBQA03274D1, SBQA05406D1, SBQA00960D1, SBQA00047D1, SBQA03772D1, SBQA03862D1 |

Table 2

| Polypeptide SEQ ID NO: | Amino Acid Residues | Potential Phosphorylation Sites | Potential Glycosylation Sites | Signature Sequences, Motifs, and Domains | Identification | Analytical Methods |
|---|---|---|---|---|---|---|
| 1 | 92 | S18 | | small proline-rich protein repeats (PR00021) | skin-specific protein (GI 2589188) | BLAST MOTIFS PRINTS |
| 2 | 118 | S85 T17 T26 | | Glycosaminoglycan attachment site: S70-G73 small proline-rich protein repeats (PR00021) | skin-specific protein (GI 2589188) | BLAST MOTIFS PRINTS |
| 3 | 110 | T21 | | Glycosaminoglycan attachment site: S66-G69 small proline-rich protein repeats (PR00021) | skin-specific protein (GI 2589188) | BLAST MOTIFS PRINTS |
| 4 | 128 | S68 T122 | | Signal peptide: M1-C20 cysteine-rich keratin associated protein motifs: C19-P22; C30-P33 C58-P61; C63-P66 C73-P76; C98-P101 C108-P111 | sheep keratin high sulfur matrix protein IIIA3 (GI 71384) | BLAST MOTIFS SPSCAN |

Table 2 (cont.)

| Polypeptide SEQ ID NO: | Amino Acid Residues | Potential Phosphorylation Sites | Potential Glycosylation Sites | Signature Sequences, Motifs, and Domains | Identification | Analytical Methods |
|---|---|---|---|---|---|---|
| 5 | 342 | T175 S234 S209 S274 S314 S318 T27 T60 T194 T211 S261 | N119 N125 | Signal peptide: M1-G63<br>SH3 domain: I286-N342 A290-D308; R328-T340 I286-G296; G300-K315 H319-R328; Q330-N342 | mouse Ray (GI 1944389) | BLAST MOTIFS SPSCAN PFAM BLOCKS PRINTS |
| 6 | 529 | T37 S50 T287 S498 S3 T57 S80 S105 S182 S395 S440 S498 | N172 | Signal peptide: M1-A32 | human S protein (GI 414810) | BLAST MOTIFS SPSCAN |

Table 3

| Nucleotide SEQ ID NO: | Selected Fragments of Nucleic Acid Sequence | Tissue Expression (Fraction of Total) | Disease or Condition (Fraction of Total) | Vector |
|---|---|---|---|---|
| 7 | 146-172 | Dermatologic (0.400)<br>Reproductive (0.400)<br>Hematopoietic/Immune (0.200) | Proliferating (0.400)<br>Cancer (0.200)<br>Inflammation (0.200) | PSPORT1 |
| 8 | 173-199 | Dermatologic (0.500)<br>Reproductive (0.250)<br>Hematopoietic/Immune (0.250) | Proliferating (0.500)<br>Inflammation (0.250)<br>Cancer (0.250) | pINCY |
| 9 | 148-174 | Dermatologic (0.500)<br>Gastrointestinal (0.500) | Proliferating (0.500)<br>Inflammation (0.500) | pINCY |
| 10 | 56-116 | | Proliferating (1.000)<br>Inflammation (0.500) | pINCY |
| 11 | 860-907 | Reproductive (0.350)<br>Nervous (0.190)<br>Cardiovascular (0.100)<br>Urologic (0.100) | Cell Proliferation and Cancer (0.640)<br>Inflammation (0.330) | pINCY |
| 12 | 646-754 | Dermatologic (0.300)<br>Gastrointestinal (0.300)<br>Endocrine (0.200)<br>Cardiovascular (0.100)<br>Reproductive (0.100) | Cell Proliferation and Cancer (0.600)<br>Inflammation (0.400) | pINCY |

Table 4

| Nucleotide SEQ ID NO: | Clone ID | Library | Library Comment |
|---|---|---|---|
| 7 | 2024646 | KERANOT02 | This library was constructed using RNA isolated from epidermal breast keratinocytes (NHEK). NHEK (Clontech #CC-2501) is a human breast keratinocyte cell line derived from a 30-year-old black female during breast-reduction surgery. |
| 8 | 3431776 | SKINNOT04 | This library was constructed using RNA isolated from breast skin tissue removed from a 70-year-old Caucasian female during a breast biopsy and resection. |
| 9 | 1798487 | COLNNOT27 | This library was constructed using RNA isolated from diseased cecal tissue removed from a 31-year-old Caucasian male during a total intra-abdominal colectomy, appendectomy, and permanent ileostomy. Pathology indicated severe active Crohn's disease involving the colon from the cecum to the rectum. There were deep rake-like ulcerations which spared the intervening mucosa. The ulcers extended into the muscularis, and there was transmural inflammation. Patient history included an irritable colon. Previous surgeries included a colonoscopy. |
| 10 | 1448744 | PLACNOT02 | This library was constructed using RNA isolated from the placental tissue of a Hispanic female fetus, who was prematurely delivered at 21 weeks' gestation. Serologies of the mother's blood were positive for CMV (cytomegalovirus). |
| 11 | 2737275 | OVARNOT09 | This library was constructed using RNA isolated from ovarian tissue removed from a 28-year-old Caucasian female during a vaginal hysterectomy and removal of the fallopian tubes and ovaries. Pathology indicated multiple follicular cysts ranging in size from 0.4 to 1.5 cm in the right and left ovaries, chronic cervicitis and squamous metaplasia of the cervix, and endometrium in weakly proliferative phase. Family history included benign hypertension, hyperlipidemia, and atherosclerotic coronary artery disease. |

Table 4 (cont.)

| Nucleotide SEQ ID NO: | Clone ID | Library | Library Comment |
|---|---|---|---|
| 12 | 3325323 | PTHYNOT03 | This library was constructed using RNA isolated from the left parathyroid tissue of a 69-year-old Caucasian female during a partial parathyroidectomy. Pathology indicated hyperplasia. The patient presented with primary hyperparathyroidism. |

# Table 5

| Program | Description | Reference | Parameter Threshold |
|---|---|---|---|
| ABI FACTURA | A program that removes vector sequences and masks ambiguous bases in nucleic acid sequences. | Perkin-Elmer Applied Biosystems, Foster City, CA. | |
| ABI/PARACEL FDF | A Fast Data Finder useful in comparing and annotating amino acid or nucleic acid sequences. | Perkin-Elmer Applied Biosystems, Foster City, CA; Paracel Inc., Pasadena, CA. | Mismatch <50% |
| ABI AutoAssembler | A program that assembles nucleic acid sequences. | Perkin-Elmer Applied Biosystems, Foster City, CA. | |
| BLAST | A Basic Local Alignment Search Tool useful in sequence similarity search for amino acid and nucleic acid sequences. BLAST includes five functions: blastp, blastn, blastx, tblastn, and tblastx. | Altschul, S.F. et al. (1990) J. Mol. Biol. 215:403-410; Altschul, S.F. et al. (1997) Nucleic Acids Res. 25: 3389-3402. | *ESTs*: Probability value= 1.0E-8 or less<br>*Full Length sequences*: Probability value= 1.0E-10 or less |
| FASTA | A Pearson and Lipman algorithm that searches for similarity between a query sequence and a group of sequences of the same type. FASTA comprises as least five functions: fasta, tfasta, fastx, tfastx, and ssearch. | Pearson, W.R. and D.J. Lipman (1988) Proc. Natl. Acad Sci. 85:2444-2448; Pearson, W.R. (1990) Methods Enzymol. 183: 63-98; and Smith, T.F. and M. S. Waterman (1981) Adv. Appl. Math. 2:482-489. | *ESTs*: fasta E value=1.06E-6<br>*Assembled ESTs*: fasta Identity= 95% or greater and Match length=200 bases or greater; fastx E value=1.0E-8 or less<br>*Full Length sequences*: fastx score=100 or greater |
| BLIMPS | A BLocks IMProved Searcher that matches a sequence against those in BLOCKS and PRINTS databases to search for gene families, sequence homology, and structural fingerprint regions. | Henikoff, S and J.G. Henikoff, Nucl. Acid Res., 19:6565-72, 1991. J.G. Henikoff and S. Henikoff (1996) Methods Enzymol. 266:88-105; and Attwood, T.K. et al. (1997) J. Chem. Inf. Comput. Sci. 37: 417-424. | Score=1000 or greater; Ratio of Score/Strength = 0.75 or larger; and Probability value= 1.0E-3 or less |
| PFAM | A Hidden Markov Models-based application useful for protein family search. | Krogh, A. et al. (1994) J. Mol. Biol., 235:1501-1531; Sonnhammer, E.L.L. et al. (1988) Nucleic Acids Res. 26:320-322. | Score=10-50 bits, depending on individual protein families |

## Table 5 (cont.)

| Program | Description | Reference | Parameter Threshold |
|---|---|---|---|
| ProfileScan | An algorithm that searches for structural and sequence motifs in protein sequences that match sequence patterns defined in Prosite. | Gribskov, M. et al. (1988) CABIOS 4:61-66; Gribskov, et al. (1989) Methods Enzymol. 183:146-159; Bairoch, A. et al. (1997) Nucleic Acids Res. 25: 217-221. | Score= 4.0 or greater |
| Phred | A base-calling algorithm that examines automated sequencer traces with high sensitivity and probability. | Ewing, B. et al. (1998) Genome Res. 8:175-185; Ewing, B. and P. Green (1998) Genome Res. 8:186-194. | |
| Phrap | A Phils Revised Assembly Program including SWAT and CrossMatch, programs based on efficient implementation of the Smith-Waterman algorithm, useful in searching sequence homology and assembling DNA sequences. | Smith, T.F. and M. S. Waterman (1981) Adv. Appl. Math. 2:482-489; Smith, T.F. and M. S. Waterman (1981) J. Mol. Biol. 147:195-197; and Green, P., University of Washington, Seattle, WA. | Score= 120 or greater; Match length= 56 or greater |
| Consed | A graphical tool for viewing and editing Phrap assemblies | Gordon, D. et al. (1998) Genome Res. 8:195-202. | |
| SPScan | A weight matrix analysis program that scans protein sequences for the presence of secretory signal peptides. | Nielson, H. et al. (1997) Protein Engineering 10:1-6; Claverie, J.M. and S. Audic (1997) CABIOS 12: 431-439. | Score=5 or greater |
| Motifs | A program that searches amino acid sequences for patterns that matched those defined in Prosite. | Bairoch et al. supra; Wisconsin Package Program Manual, version 9, page M51-59, Genetics Computer Group, Madison, WI. | |

**Claims**

1. A substantially purified polypeptide comprising an amino acid sequence selected from the group consisting of SEQ ID NOs: 4 and 5, SEQ ID NOs: 1-3 and 6 and fragments thereof.

2. A substantially purified variant having at least 90% amino acid identity to the amino acid sequence of claim 1.

3. An isolated and purified polynucleotide encoding the polypeptide of claim 1.

4. An isolated and purified polynucleotide variant having at least 70% polynucleotide sequence identity to the polynucleotide of claim 3.

5. An isolated and purified polynucleotide which hybridizes under stringent conditions to the polynucleotide of claim 3.

6. An isolated and purified polynucleotide having a sequence which is complementary to the polynucleotide sequence of claim 3.

7. An isolated and purified polynucleotide comprising a polynucleotide sequence selected from the group consisting of SEQ ID NOs: 10 and 11, SEQ ID NOs: 7-9 and 12 and fragments thereof.

8. An isolated and purified polynucleotide variant have at least 70% polynucleotide sequence identity to the polynucleotide of claim 7.

9. An isolated and purified polynucleotide having a sequence which is complementary to the polynucleotide of claim 7.

10. An expression vector comprising at least a fragment of the polynucleotide of claim 3.

11. A host cell comprising the expression vector of claim 10.

12. A method for producing a polypeptide comprising the amino acid sequence selected from the group consisting of SEQ ID NOs: 4 and 5, SEQ ID NOs: 1-3 and 6 and fragments thereof, the method comprising the steps of:

    (a) culturing the host cell of claim 11 under conditions suitable for the expression of the polypeptide; and
    (b) recovering the polypeptide from the host cell culture.

13. A pharmaceutical composition comprising the polypeptide of claim 1 in conjunction with a suitable pharmaceutical carrier.

14. A purified antibody which specifically binds to the polypeptide of claim 1.

15. A purified agonist of the polypeptide of claim 1.

16. A purified antagonist of the polypeptide of claim 1.

17. A method for treating or preventing a disorder associated with decreased expression or activity of HEPI, the method comprising administering to a subject in need of such treatment an effective amount of the pharmaceutical composition of claim 13.

18. A method for treating or preventing a disorder associated with increased expression or activity of HEPI, the method comprising administering to a subject in need of such treatment an effective amount of the antagonist of claim 16.

19. A method for detecting a polynucleotide encoding the polypeptide comprising the amino acid sequence selected from the group consisting of SEQ ID NOs: 4 and 5, SEQ ID NOs: 1-3 and 6 and fragments thereof in a biological sample, the method comprising the steps of:

    (a) hybridizing the polynucleotide of claim 6 to at least one of the nucleic acids in the biological sample, thereby forming a hybridization complex; and
    (b) detecting the hybridization complex, wherein the presence of the hybridization complex correlates with the presence of the polynucleotide encoding the polypeptide in the biological sample.

**20.** The method of claim 19 further comprising amplifying the polynucleotide prior to hybridization.

```
1    MSCQQNQQQCQPPPKC--------PSPKCP  2024646
1    MSCQQSQQQCQPPPKCTPKCPPKCPTPKCP  3431776
1    MSCQQNQQQCQPPPKCPPKCTPKCP-PKCP  1798487
1    MSCQQNQQQCQPPPKCPPKCTPKCP-PKCP  GI 2589188

23   PKSPVQCLPPAS---------------SGC  2024646
31   PKCPPKCPPVSSCCSVSSGGCCGSSSGGSC  3431776
30   PKCPPQCPAP---CFPAVSSCCGPSSGSCC  1798487
30   PKCLPQCPAP---CSPAVSSCCGPISGGCC  GI 2589188

38   APSSGVCGPSSEGGCFLNHHRRH--HRCRR  2024646
61   GSSSGGCCSSGGGGCCLSHHRRRRSHCHRP  3431776
57   GPSSGGCCSSGAGGCSLSHHRPRLFHRRRH  1798487
57   GPSSGGCCNSGAGGCCLSHHRPRLFHRRRH  GI 2589188

66   QRPNSCDRGSGQQGGGSGCC-----HGSGG  2024646
91   QSSGCC----SQPSGGSSCCGGGSGQHSGG  3431776
87   QSPDCCE---SEPSGGSGCC-----HSSGG  1798487
87   QSPDCCE---SEPSGGSGCC-----HSSGG  GI 2589188

91   CC                              2024646
117  CC                              3431776
109  CC                              1798487
109  CC                              GI 2589188
```

FIGURE 1

```
1    M T G S C C G S T L S S L S Y G G G C C Q P C C C R Y P C C  1448744
1    - T G S C C G P T F S S L S C G G G C L Q P R Y Y R D P C C  GI 71384

31   C R P V T C Q T T V C R P V T C V P R C T R P I C E P C C R  1448744
30   C R P V S C Q T - V S R P V T F V P R C T R P I C E P C R R  GI 71384

61   P V C C D P C S L Q E G C C R P I T C C P S S C T A V V C R  1448744
59   P V C C D P C S L Q E G C C R P I T C C P T S C Q A V V C R  GI 71384

91   P C C W A T T C C Q P V S V Q S P C C R P P C G Q P T P C S  1448744
89   P C C W A T T C C Q P V S V Q C P C C R P T S C Q P A P C S  GI 71384

121  - T T C R T - - - - S S C                                    1448744
119  R T T C R T F R T S P C C                                    GI 71384
```

FIGURE 2

```
1    MNNPIPSNLKSEAKKAAKILREFTEITSRN  2737275
1    MNNPIPSNLKSEAKKAAKILREFTEITSRN  GI 1944389

31   GPDKIIPAHVIAKAKGLAILSVIKAGFLVT  2737275
31   GPDKIIPAHVIAKAKGLAVLSVIKAGFLVT  GI 1944389

61   ARGGSGIVVARLPDGKWSAPSAIGIAGLGG  2737275
61   ARGGSGIVLARLPDGKWSAPSAIGIAGLGG  GI 1944389

91   GFEIGIEVSDLVIILNYDRAVEAFAKGGNL  2737275
91   GFEIGIEVSDLVIILNYDRAVEAFAKGGNL  GI 1944389

121  TLGGNLTVAVGPLGRNLEGNVALRSSAAVF  2737275
121  TLGGNFTVAVGPLGRNLEGNVSLRSSAAVF  GI 1944389

151  TYCKSRGLFAGVSLEGSCLIERKETNRKFY  2737275
151  TYCKSRGLFAGISLEGSCLIERKETNRKFY  GI 1944389

181  CQDIRAYDILFGDTPRPAQAEDLYEILDSF  2737275
181  CQDIRAYDILFGDVPQPAQAEDLYEILNSF  GI 1944389

211  TEKYENEGQRINARKAAREQRKSSAKELPP  2737275
211  TEKYETEGQRINLKKVAREQRK--AKELPP  GI 1944389
```

FIGURE 3A

```
241 KPLSRPQQSSAPVQLNSGSQSNRNEYKLYP 2737275
239 KPSSRPQPAHPPVQLNAGSQGNRNEYKLYP GI 1944389

271 GLSSYHERVGNLNQPIEVTALYSFEGQQPG 2737275
269 ELSSYHEKTGNLNQPIEVTALYSFEGQQPG GI 1944389

301 DLNFQAGDRITVISKTDSHFDWWEGKLRGQ 2737275
299 DLNFQAGDRIIVISKTDSNFDWWEGKLRGQ GI 1944389

331 TGIFPANYVTMN                   2737275
329 TGIFPANYVTMN                   GI 1944389
```

FIGURE 3B

```
1    MGSSRAPWMGRVGGHGMLALLLAGLLLPGT 3325323
1    ---------------MLALLLAGLLLPGT GI 414810

31   LAKSIGTFSDPCKDPTRITSPNDPCLTGKG 3325323
15   LAKSIGTFSDPCKDPTRITSPNDPCLTGKG GI 414810

61   DSSGFSSYSGSSSSGSSISSARSSGGGSSG 3325323
45   DSSGFSSYSGSSSSGSSISSARSSGGGSSG GI 414810

91   SSSGSSIAQGGSAGSFKPGTGYSQVSYSSG 3325323
75   SSSGSSIAQGGSAGSFKPGTGYSQVSYSSG GI 414810

121  SGSSLQGASGSSQLGSSSSHSGSSGSHSGS 3325323
105  SGSSLQGASGSSQLGSSSSHSGSSGSHSGS GI 414810

151  SSSHSSSSSSFQFSSSSFQVGNGSALPTND 3325323
135  SSSHSSSSSSFQFSSSSFQVGNGSALPTND GI 414810

181  NSYRGILNPSQPGQSSSSSQTFGVSSSGQS 3325323
165  NSYRGILNPSQPGQSSSSSQTSGVSSSGQS GI 414810

211  VSSNQRPCSSDIPDSPCSGGPIVSHSGPYI 3325323
195  VSSNQRPCSSDIPDSPCSGGPIVSHSGPYI GI 414810
```

FIGURE 4A

```
241 PSSHSVSGGQRPVVVVVDQHGSGAPGVVQG 3325323
225 PSSHSVSGGQRPVVVVVDQHGSGAPGVVQG GI 414810

271 PPCSNGGLPGKPCPPITSVDKSYGGYEVVG 3325323
255 PPCSNGGLPGKPCPPITSVDKSYGGYEVVG GI 414810

301 GSSDSYLVPGMTYSKGKIYPVGYFTKENPV 3325323
285 GSSDSYLVPGMTYSKGKIYPVGYFTKENPV GI 414810

331 KGSPGVPSFAAGPPISEGKYFSSNPIIPSQ 3325323
315 KGSPGVPSFAAGPPISEGKYFSSNPIIPSQ GI 414810

361 SAASSAIAFQPVGTGGVQLCGGGSTGSKGP 3325323
345 SAASSAIAFQPVGTGGVQLCGGGSTGSKGP GI 414810

391 CSPSSSRVPSSSSISSSSGLPYHPCGSASQ 3325323
375 CSPSSSRVPSSSSISSSSGSPYHPCGSASQ GI 414810

421 SPCSPPGTGSFSSSSSSQSSGKIILQPCGS 3325323
405 SPCSPPGTGSFSSSSSSQSSGKIILQPCGS GI 414810

451 KSSSSGHPCMSVSSLTLTGGPDGSPHPDPS 3325323
435 KSSSSGHPCMSVSSLTLTGGPDGSPHPDPS GI 414810
```

FIGURE 4B

```
481 A G A K P C G S S S A G K I P C R S I R D I L A Q V K P L G 3325323
465 A G A K P C G S S S A G K I P C R S I R - - - - - - - - - - GI 414810

511 P Q L A D P E V F L P Q G E L L D S P                       3325323
485 - - - - - - - - - - - - - - - - - I S                       GI 414810
```

FIGURE 4C